# EUROPÄISCHE PATENTSCHRIFT

(11) **EP 3 217 975 B1**
(45) Veröffentlichungstag und Bekanntmachung des Hinweises auf die Patenterteilung: **11.03.2020**
(21) Anmeldenummer: 15801987.7
(22) Anmeldetag: 08.10.2015
(51) Int. Cl.: A61K 31/423, A61K 31/5575, A61K 35/744, A61K 38/19, A61P 35/02, A61P 37/02

(54) **VERWENDUNG VON IMMUNMODULATORISCH WIRKSAMEN ZUSAMMENSETZUNGEN ZUR IMMUNTHERAPEUTISCHEN BEHANDLUNG VON PATIENTEN MIT MYELOISCHEN LEUKÄMIEN**
USE OF IMMUNOMODULATORY EFFECTIVE COMPOSITIONS FOR THE IMMUNOTHERAPEUTIC TREATMENT OF PATIENTS SUFFERING FROM MYELOID LEUKEMIAS
UTILISATION DE COMPOSITIONS À EFFET IMMUNOMODULATEUR POUR UN TRAITEMENT IMMUNOTHÉRAPEUTIQUE DE PATIENTS ATTEINTS DE LEUCÉMIES MYÉLOÏDES

(30) Priorität: 09.10.2014 DE 102014014993
(43) Veröffentlichungstag der Anmeldung: 20.09.2017
(73) Patentinhaber: Schmetzer, Helga, 82041 Oberhaching (DE)
(72) Erfinder: Schmetzer, Helga, 82041 Oberhaching (DE)
(86) Internationale Anmeldenummer: PCT/EP2015/001979
(87) Internationale Veröffentlichungsnummer: WO 2016/055161

(56) Entgegenhaltungen:
- WO-A1-2011/068896
- US-A- 6 010 905
- H. RODDIE ET AL: "Phase I/II study of vaccination with dendritic-like leukaemia cells for the immunotherapy of acute myeloid leukaemia", BRITISH JOURNAL OF HAEMATOLOGY, Bd. 133, Nr. 2, April 2006 (2006-04), Seiten 152-157, XP055247786, GB ISSN: 0007-1048, DOI: 10.1111/j.1365-2141.2006.05997.x
- GRABRUCKER C ET AL: "The quality and quantity of leukemia-derived dendritic cells from patients with acute myeloid leukemia and myelodysplastic syndrome are a predictive factor for the lytic potential of dendritic cells-primed leukemia-specific T cells", JOURNAL OF IMMUNOTHERAPY 2010 LIPPINCOTT WILLIAMS AND WILKINS USA, Bd. 33, Nr. 5, Juni 2010 (2010-06), Seiten 523-537, XP009188390, ISSN: 1524-9557 in der Anmeldung erwähnt
- KREMSER A ET AL: "Dendritic cells (DCs) can be successfully generated from leukemic blasts in individual patients with AML or MDS: An evaluation of different methods", JOURNAL OF IMMUNOTHERAPY 2010 LIPPINCOTT WILLIAMS AND WILKINS USA, Bd. 33, Nr. 2, Februar 2010 (2010-02), Seiten 185-199, XP009188389, ISSN: 1524-9557 in der Anmeldung erwähnt
- SCHICK, JULIA: "PRäDIKTIVE RELEVANZ REGULATORISCHER T-ZELLSUBPOPULATIONEN NACH STIMULATION MIT DENDRITISCHEN ZELLEN LEUKäMISCHER ABSTAMMUNG FüR DIE ANTILEUKäMISCHE T-ZELLANTWORT", DISSERTATION LMU MÜCHEN, 3 April 2014 (2014-04-03),
- CHRISTIAN M. SCHÜRCH ET AL: "DENDRITIC CELL-BASED IMMUNOTHERAPY FOR MYELOID LEUKEMIAS", FRONTIERS IN IMMUNOLOGY, vol. 4, 2013, pages 1-16,
- WEIGEL BRENDA J ET AL: "Comparative analysis of murine marrow-derived dendritic cells generated by Flt3L or GM-CSF/IL-4 and matured with immune stimulatory agents on the in vivo induction of antileukemia responses", BLOOD, AMERICAN SOCIETY OF HEMATOLOGY, US, vol. 100, no. 12, 8 August 2002 (2002-08-08), pages 4169-4176, XP002510254, ISSN: 0006-4971, DOI: 10.1182/BLOOD-2002-04-1063

## Beschreibung

### I.Technisches Gebiet der Erfindung

Immuntherapeutische Behandlung von Patienten mit myeloischen Leukämien.

Die vorliegende Erfindung betrifft verschiedene Zusammensetzungen aus verschiedenen immunmodulatorisch wirksamen Substanzen, zur Verwendung in der Behandlung von Patienten mit myeloischen Leukämien. Ausserdem betrifft die vorliegende Erfindung ein Verfahren zur Auswahl einer geeigneten Zusammensetzung/Wirkstoffkombination zur Verwendung in der Behandlung von Patienten mit myeloischen Leukämien.

### II.Hintergrund der Erfindung

**KLINIK:** Myeloische Leukämien wie die akute myeloische Leukämie (AML), Myelodysplasien (MDS) oder chronisch myeloische Leukämien (CML) sind klonale hämopoetische Erkrankungen, die bei pathologisch gesteigerter Zellproliferation und gestörter Differenzierung der Immunabwehr entgehen. Je nach Subtyp und insbesondere therapierefraktäre/rezidivierte Stadien gehen diese Erkrankungen mit einer schlechten Prognose einher; ferner erleiden ca 80% erfolgreich chemotherapierter Patienten in den folgenden zwei Jahren einen Rückfall ihrer Erkrankung. Daher besteht ein dringender Bedarf an innovativen immuntherapeutischen Behandlungsansätzen (Reagan: Cellular immunotherapy tor refractory hematological malignancies. J Transl Med. 19,11,150 2013). Die allogene Stammzelltransplantation (SZT) gilt als die wirkungsvollste antileukämische Therapie, jedoch vor allem für jüngere Patienten.. Neben der intensiven (Radio)-Chemotherapie gilt hierbei der allogene Graftversus-Leukemia (GvL) Immuneffekt als tragendes therapeutisches Prinzip. Nach heutigem Kenntnisstand wird der GvL-Effekt in erster Linie durch T-Lymphozyten des Spenders vermittelt. Komplikationen nach Therapie, verursacht durch die fehlende Regeneration/Immunkompetenz von Blutzellen, sind Infekte, Blutungsneigung, Anämien oder Rezidive, verursacht durch wiederkehrende Blasten. Bei Patienten nach SZT können, verursacht durch immunreaktive Zellen des Stammzellspenders akute autoimmune Reaktionen gegen Empfängerzellen auftreten (,Graft versus Host' (GvH) Reaktionen). Dabei kann es zu schweren bis lebensbedrohlichen entzündlichen Reaktionen u.a. gegen Gefäßsysteme kommen, wie z.B bei der Veno-occlussive Verschlusskrankheit (VOD). Neben T-Zellen spielen v.a dendritische Zellen (DC) als professionelle T-Zellstimulatoren bei der Vermittlung von Immunreaktionen eine entscheidende Rolle. Grundsätzlich konnten bereits therapeutische Effekte (Blastenreduktion) nach Vakzinierungen von AML-Patienten mit Antigen (z.B.WT1) -gepulsten DC gezeigt werden. Diese Strategie bleibt allerdings nur Patienten mit definiertem HLA-Typ (meist HLA A2) und Kenntnis eines leukämie-assoziierten Antigens (z.B WT1) vorbehalten.

Die im Vergleich zu lymphatischen Leukämieformen besseren Ansprechraten myeloischer Leukämien (CML, AML, MDS) wurden auf die Fähigkeit der Blasten zur Differenzierung zu myeloischen 'leukemia-derived' DC (DCₗₑᵤ) und verbesserten Präsentation Leukämie-assoziierter Antigene zurückgeführt (Schmid : Low-dose ARAC, donor cells, and GM-CSF for treatment of recurrent acute myeloid leukemia after allogeneic stem cell transplantation. Leukemia; 18(8):1430-3, 2004). Auf der Grundlage dieses Konzepts wurde in der Behandlung des AML-Rezidivs nach allogener SZT GM-CSF bei einigen Patienten systemisch eingesetzt, um eine Differenzierung myeloischer Blasten zu antigenpräsentierenden Zellen in vivo zu induzieren. (Stand der Technik ist, dass Zytokine wie GM-CSF oder Kit-Ligand alleine oder in Kombination mit anderen stammzellaktiven Zytokinen (z.B G-CSF) oder diversen small molecules als 'Stammzellregenrations-Zytokine' Patienten verabreicht werden mit dem Ziel, dass der Körper Stammzellen aus dem Knochenmark ins Blut mobilisiert und somit Blutzellen (z.B nach Chemotherapie oder Organschädigung) regeniert (**Besmer**: Composition of cKitligand, GM-CSF and TNFa and Method of use, US Patent Nr 6,001,803 1999).

**DC/DCₗₑᵤ-GENERIERUNG EX VIVO:** Eine Reihe verschiedener Methoden sind zur ex *vivo Differenzierung von DC* bei AML-Patienten geeignet, jedoch ist nicht mit allen Methoden bei jedem einzelnen Fall eine erfolgreiche Generierung möglich ist. Es wurde eine Strategie entwickelt, mit der besten von drei vorab getesteten Methoden (CA, MCM, PICI, INTR) bei jedem einzelnen Fall DC (v.a. reife DC und DCₗₑᵤ) in der Zellkultur zu erhalten (Kremser/ Schmetzer: Dendritic cells can be successfully generated from leukemic blasts in individual patients with AML or MDS. J. Immunotherapy 33:185-199, 2010); Dreyssig/Schmetzer: Various 'Dendritic Cell Antigens' are already expressed on uncultured Blasts in Acute Myeloid Leukemia and Myelodysplastic Syndromes. Immunotherapy 3(9), 1113-1124 (2011); Schick/Schmetzer: Antileukemic T-cell responses can be predicted by the composition of specific regulatory T-cell subpopulations. J. Immunotherapy, Vol.36, 4, 223-237; (2013) **Tabelle 1a** (Spalten a1-a4) zeigt die genannten Methoden: sie enthalten zahlreiche Wirkstoffe in Kombination: verschiedene Zytokine und 'Danger- und Maturierungsfaktoren', die eine DC-Differenzierung aus Monozyten oder Blasten (zunächst in der serumfreien Zellkultur aus mononukleären Zellen (MNC)) induzieren. Dabei wurden in jeder Methode jeweils die in **Tabelle 1a** angegebenen Wirkstoffe in Kombination verwendet. Die leukämische Abstammung der DC wurde durch Nachweis der klonalen leukämie-assoziierten Chromosomenaberrationen IN den DC durch Fluoreszenz In situ Hybridisierung (FISH) oder durch den durchflusszytometrischen Nachweis des individuellen Blasten-Antigenmusters auf der Zelloberfläche der DC belegt.

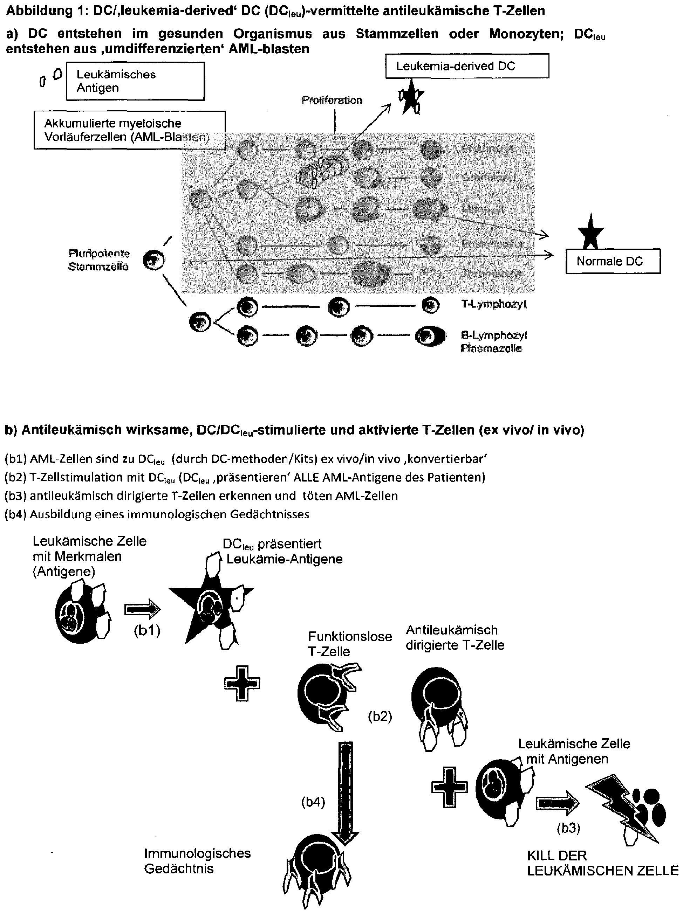

Der Vorteil einer Herstellung von DC direkt aus leukämischen Blasten ist, dass Tumorantigene nicht bekannt sein müssen, da die Blasten zu DCₗₑᵤ konvertiert werden und dabei das gesamte leukämische Antigenspektrum jedes Patienten präsentieren. Mit Hilfe dieser Methoden konnte gezeigt werden, dass keine der DC-Generierungsmethoden eine Reinkultur an DCₗₑᵤ hervorbringt: ca 45-55% der erhaltenen DC sind nachweislich leukämischer Abstammung, andererseits sind ca 50-60% der vorhandenen Blasten konvertierbar zu DCₗₑᵤ. Das heißt, dass zwar regelmäßig DC leukämischer Abstammung generiert, aber nicht alle Blasten in DC konvertiert werden. **Abbildung 1a** zeigt schematisch am Beispiel AML, dass neben normalen DC (aus Monozyten oder Stammzellen) auch DCₗₑᵤ entstehen, d.h. dass Blasten zu DC 'umdifferenziert' werden

**T-ZELLSTIMULATION MIT DC/DCₗₑᵤ EX VIVO:** Durch ex *vivo Stimulationsversuche von T-Zellen mit DC*/*DCₗₑᵤ* können anti-leukämische T-Zellen vermehrt (im Vergleich zu unstimulierten oder Blasten-stimulierten T-Zellen) entstehen (Grabrucker/Schmetzer: The Quality and Quantity of Leukemia-Derived Dendritic Cells (DC) from Patients with Acute Myeloid Leukaemia and Myelodysplastic Syndrom are a Predictive Factor for the Lytic Potential of DC-primed Leukemia-specific T-Cells J Immunotherapy 33,5,523-537, 2010). **Abbildung 1b** zeigt exemplarisch den Versuchsaufbau für derartige Stimulationsversuche in gemischten Lymphozytenkulturen (MLC): myeloische leukämische Zellen (z.B AML-Zellen) sind zu DCₗₑᵤ (durch DC-methoden/Kits) 'konvertierbar' (**b1**). Diese DCₗₑᵤ 'präsentieren' alle AML-Antigene des Patienten und stimulieren dadurch T-Zellen (**b2**), die wiederum AML-Zellen erkennen und töten können (**b3**). Ferner wird ein immunologisches Gedächtnis angelegt (**b4**). Durch T-Zellstimulationsversuche in gemischten Lymphozytenkulturen (MLC) konnte in der Vergangenheit gezeigt werden, dass DC-stimulierte T-Zellen nicht nur besser als blastenstimulierte bzw. unstimulierte T-Zellen proliferieren, sondern auch häufiger und effektiver eine leukämiezytotoxische Wirksamkeit (wenngleich auch nicht in jedem Fall) entfalten. Dabei ist sowohl die *Qualität* der DC (z.B. Reifegrad, Anteile an ,leukemia-derived DC', etc.), als auch die *Zusammensetzung der resultierenden T-Zellen* (z.B. Anteile an CD4 bzw. nicht-naiven CD45RO⁺ Zellen, CD4:CD8 Verhältnisse, regulatorischen, β-Integrin-exprimierenden T-Zellen, etc.) sowie *lösliche Faktoren im Zellkulturüberstand* (z.B: IFNγ,IL-6,CXCL8,CCL2) prädiktiv für die Funktion DC-stimulierter T-Zellen sowie für das Ansprechen von Patienten auf Immuntherapien (SCT oder DLI-therapie). In T-*Zell-Kionalitätsanalysen* konnte gezeigt werden, dass nach 'DC/DCₗₑᵤ'- im Vergleich zu Blasten-Stimulation das T-Zellrepertoire eingeschränkt wird: manche CD4/CD8 Klone waren bevorzugt klonal vermehrt, was dafür spricht, dass DC/DCₗₑᵤ Immunreaktionen **"dirigieren, fokussieren und verstärken"'.** Interessant war, dass sich durch Spektratyping in Kombination mit Sequenzanalysen ein *identischer T-Zellklon* (identische T-Zellrezeptor-Vß-sequenz) nach DC-Stimulation ex vivo sowie in vivo aus T-Zellen eines Patienten nach SCT nachweisen ließ, was dafür spricht, dass mit DC-Kulturen in vivo Vorgänge nicht nur simuliert, sondern diese Klone auch funktionell analysiert werden können (z.B. durch funktionellen Blastenlysetest).

Da die antileukämische Funktion der T-Zellen im Körper von AML-Patienten für die Erhaltung einer langanhaltenden Remission entscheidend ist wäre eine Optimierung der antileukämischen T-Zell-Funktion sowie die Generierung von T-Gedächtniszellen (die jederzeit spezifische Effektorzellen bereitstellen können) von großem klinischen Nutzen zur Bekämpfung (residueller) leukämischer Zellen im Körper der Patienten.

Unklar ist ob nach DC-Stimulation eine Expansion (vieler oder weniger) spezifischer antileukämischer T-Zellpopulationen (in vivo, in vitro) stattfindet, ob ein immunologisches Gedächtnis angelegt wird, ob die Funktion spezifischer T-Zellen durch lösliche Faktoren beeinflusst wird und vor allem, ob die antileukämische Funktion in vivo ausreicht, um Remissionen zu erzielen bzw aufrecht zu erhalten. Daher muss begleitend zu Immuntherapien ein gezieltes *'Monitoring'* (Identifizierung, Quantifizierung) relevanter T-Zellpopulationen ex vivo bzw in vivo durch Einsatz geeigneter Methoden (Durchflußzytometrie, Molekularbiologie) erfolgen.

Grabrucker et al, J Immunotherapy 2010, 33,5,523-537, beschreibt die Differenzierung von DC mit Wirkstoffkombinationen.

### III. Zusammenfassung der Erfindung

Die Aufgabe der vorliegenden Erfindung besteht darin, Blasten von Patienten mit myeloischen Leukämien mit immunmodulierenden Verfahren *in vivo* so zu modifizieren, dass sie zu einer 'Vakzine' werden, die immunreaktive Zellen (des Patienten oder des Stammzellspenders) gezielt gegen Blasten aktivieren kann. Diese Aufgabe wird durch die Merkmale der **unabhängigen Ansprüche** gelöst. Weitere Ausführungsformen der Erfindung sind in den abhängigen Ansprüchen angegeben.

Die vorliegende Beschreibung ermöglicht die alternative Verwendung von verschiedenen Wirkstoffkombinationen, zusammengesetzt aus verschiedenen immunmodulatorisch wirksamen Substanzen, zur Behandlung von Patienten mit myeloischen Leukämien - im Sinne einer personalisierten Medizin. Dabei wird zunächst in einem Kultursystem mit gerinnungsgehemmten Vollblutproben der Leukämiepatienten in blastenreichen Stadien aus 6 alternativen Kits das patientenindividuell beste ausgewählt: als bestes Kit gilt das, das die beste 'Umwandlung' von Blasten in leukemia-derived Dendritic Cells (DCₗₑᵤ) als antigenpräsentierende Zellen ohne Induktion der Blastenproliferation und zugleich die beste antileukämische T-Zellaktivierung erzielt.

### IV. Beschreibung der Abbildungen und Tabellen

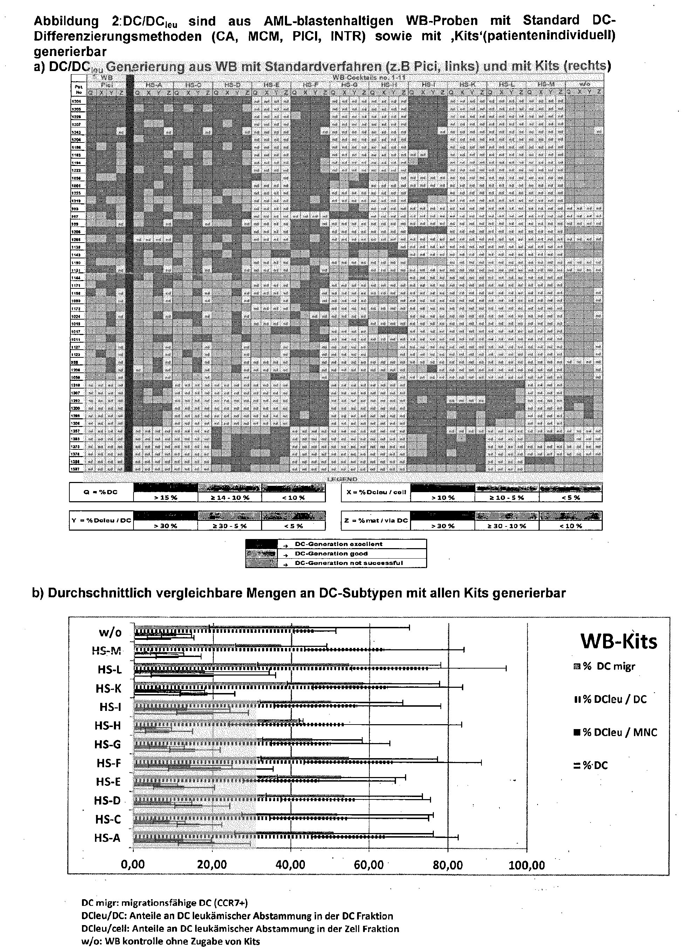

**Abbildung 1****:** DC/'leukemia-derived' DC (DCₗₑᵤ)-vermittelte antileukämische T-Zellen
   a) DC entstehen im gesunden Organismus aus Stammzellen oder Monozyten; DCₗₑᵤ entstehen aus 'umdifferenzierten' AML-blasten
   b) Antileukämisch wirksame, DC/DCₗₑᵤ-stimulierte und aktivierte T-Zellen (ex vivo/ in vivo) **Abbildung 2****:** DC/DCₗₑᵤ sind aus AML-blastenhaltigen WB-Proben mit Standard DC-Differenzierungsmethoden (z.B: PICI) sowie mit 'Kits' (patientenindividuell) generierbar
   a) DC/DCₗₑᵤ Generierung aus WB mit Standardverfahren (z.B Pici, links) und mit Kits (rechts)
   b) Durchschnittlich vergleichbare Mengen an DC-Subtypen mit allen Kits
**Abbildung 3****:** Auswahl der besten WB-Kitmethode zur Generierung von DC/DCₗₑᵤ-subtypen bei individuellen AML-Patienten: Dargestellt sind die prozentualen Häufigkeiten einer erfolgreichen DC/DCₗₑᵤ-Generierung mit individuellen Kits
   a) RANKING: 'beste oder zweitbeste DC-Methode' im Vergleich zu mindestens 3 anderen Kits
   b) RANKING: DC-Methode mit 'excellent oder high quality' (Kriterien siehe 3c).
   c) Kriterien einer erfolgreichen DC-Generierung
   d) Auswahl der 6 besten Kits nach RANKING -Kriterien
**Abbildung 4****:** Antileukämisch-blastenlytische Aktivität von T-Zellen, die mit KITBEHANDELTEN (HS-D,F,I,M,K,A) im Vergleich zu unbehandelten AML-WB-Proben stimuliert wurden
   a) Auswahl nach patientenindividuell **bestem Lysewert**
   b) Auswahl nach patientenindividuell **größter Differenz** zum WB-Kontrolle-Lysewert
   c) Auswahl von **HS-D,I,K,M behandelten** WB-Proben im Vergleich zur WB-Kontrolle
**Abbildung 5****:** Behandlung von BNML-Ratten mit Kits führt zu Reduktion der Blastenmenge und Änderungen der Tzellkomposition in Blut und Milz im Vergleich zur Placebo-Kontrolle
   a) Experimentelles Design der Rattenbehandlung
   b) Blastenreduktion in Blut (links) und Milz (rechts)
   c) Anstieg von CD62L+ (Tmem): CD4+(links) und CD8+ (rechts) in Blut und Milz
   d) Reduktion von CD4+CD25+FoxP3+Treg in der Milz

**Tabelle 1:** Generierung von DC/DCₗₑᵤ aus AML-blasten-haltigen Mononukleären -(MNC) oder Vollblut (WB)-Zellen mit verschiedenen DC-Differenzierungsmethoden bzw bestimmten Kits
   a) DC-Differenzierungsmethoden bzw Kits zur Generierung von DC/DCₗₑᵤ aus blastenhaltigen MNC (a1-a4) oder WB (a1-a5)
   b) Vorteile und Wertigkeit einer DC-Generierung aus Vollblutproben
**Tabelle 2:** Wirkstoffe in den Kits zur Immunmodulation von AML-Blasten
**Tabelle 3:** Zusammensetzung der getesteten Kits

### V. Ausführliche Beschreibung der Erfindung

Die im Folgenden am Beispiel der AML dargestellte Erfindung ist gleichermaßen bei Patienten mit anderen myeloischen Leukämien anwendbar. Gemäß der vorliegenden Erfindung wurde *in vitro* eine minimalisierte Kombination aus klinisch zugelassenen "Immunmodulatoren" entwickelt, geprüft und optimiert. Dazu wurde zunächst ein Versuchsmodell mit "heparinisierten Vollblut-(WB)proben" (abgenommen in Heparin-Röhrchen mit standardisierter Heparinzugabe) erarbeitet: WB enthält alle löslichen und zellulären Bestandteile des an Leukämie erkrankten Patienten und somit auch alle zellulären und löslichen Einflussgrößen auf die DC-Generierung. Somit kann auch gleichzeitig *in vitro* erarbeitet werden, ob *in vivo* eine DC-Differenzierung grundsätzlich möglich ist, d.h. ggfs. auch Differenzierungs-resistenzen der Zellen überwunden und somit eine funktionierende humorale und zelluläre Immunität im Patienten (wieder)hergestellt werden kann. Sehr zellreiche WB-Proben wurden mit ca 30-50% serumfreiem Medium (X-vivo) verdünnt, um eine bessere Vergleichbarkeit der einzelnen Fälle zu gewährleisten. **Tabelle 1b** zeigt Vorteile einer Generierung von DC/DCₗₑᵤ aus WB: alle im Körper vorliegenden Zellarten und löslichen Faktoren können in ihrer "natürlichen Umgebung" nachgewiesen werden, Funktionalitätstests nahe an der klinischen Wirklichkeit getestet werden und letztendlich eher Rückschlüsse auf biologische Zusammenhänge und Reaktionsprofile gezogen werden als bei Ergebnissen mit mononukleären Zellen, der normalerweise verwendeten Leukozytenfraktion für Untersuchungen.

Ähnliche Anteile an DC (Subtypen, durchflusszytometrisch ermittelt) wurden aus WB wie aus MNC von Leukämiepatienten mit den Standardmethoden (CA, PICI, MCM oder INTR, **Tabelle 1a**) generiert, was bedeutet, dass ein WB-Modell grundsätzlich geeignet ist, die Modulation von Blasten ex vivo zu induzieren, die grundsätzliche Konvertierbarkeit der Blasten zu DCₗₑᵤ zu überprüfen sowie auch ein gutes physiologisches Modell für Prüfungen einer erfolgreichen Immunmodulation ist. In einem nächsten Schritt wurde nachgewiesen, dass die Generierung von DC(Subtypen) aus WB mit den Standardmethoden in ähnlichem Maße wie mit minimalisierten Kits, bestehend aus 1-3 klinisch zugelassenen Immunmodulatoren, aus WB möglich ist, während Kulturkontrollen ohne Zugabe von Kits keine DCs aus den Blasten hervorbringen (auf die Zusammensetzung der Kits wird weiter unten eingegangen). **Abbildung 2a** zeigt Ergebnisse der DC-Generierung aus WB mit Standardverfahren 'Pici' (links) vergleichend zu WB mit Kits (rechts): viele der 11 getesteten Kits bringen ähnliche Mengen an DC-subtypen aus WB von Leukämiepatienten wie mit Standardmethoden hervor. Durchschnittlich (s. Balkendiagramme **Abbildung 2a** **und b**) entstehen ähnliche Anteile an DC(Subtypen (s. Bunttabelle): % DC ('Q'), reife/ migrationsfähige DC (,Z'), zu DCₗₑᵤ konvertierte Blasten, DCₗₑᵤ in der gesamten Zellfraktion (,X') bezogen auf alle DC ('Y')) aus WB: mit mindestens einer Methode war eine gute DC Generierung bei jedem Patienten möglich. Kulturkontrollen ohne Zugabe von Kits sind in der Regel erfolglos; ferner ist auch erkennbar, dass nicht jeder Kit gleichermaßen gut bei individuellen Patienten geeignet ist DC(Subtypen) zu generieren (s. Bunttabelle, **Abbildung 2a**).

Somit ist ein WB-Verfahren ein gutes physiologisches Modell für DC/DCₗₑᵤ-Generierungen, wobei patientenindividuelle Unterschiede erkennbar sind: einzelne Kits sind besser als andere geeignet DC(Subtypen) zu generieren.

Im Rahmen der vorliegenden Erfindung wurden mehrere Kits (vergleichend) geprüft. Alle Substanzen sind für die Anwendung am Menschen zugelassen und werden regelmäßig Patienten bei anderen Indikationen als hier aufgeführt eingesetzt. Eine Reihe von Einzelsubstanzen sowie ihre Kombinationen wurden dazu im Hinblick auf ihre Fähigkeiten untersucht, DC(Subtypen) zu generieren, leukämische Zellen zur Proliferation anzuregen, bzw. die erhaltenen DC-Produkte schließlich auf die Fähigkeit zur Aktivierung antileukämischer T-Zellen getestet. **Tabelle 2** zeigt zunächst die verwendeten ***Einzelwirkstoffe,*** ihre biologisch/klinische Wirkung sowie bisherige klinische Anwendungsgebiete: Zusammenfassend sei an dieser Stelle erwähnt, dass GM-CSF, IFNα, TNFa und PICIBANIL als Immunmodulatoren und Immunstimulanzien, zur hämatopoetischen Regeneration und systemisch zur Behandlung verschiedener Erkrankungen verabreicht werden (PICIBANIL wird auch lokal in Tumore appliziert). PGE₂ wird in hohen Konzentrationen zur Geburtseinleitung verwendet und dabei vaginal-lokal bzw systemisch verabreicht. PGE₁ wird wegen seiner vasodilatatorischen Eigenschaften systemisch beispielsweise zur Behandlung der VOD-Erkrankung oder von Ischämien an Extremitäten verabreicht. TNFa kann systemisch, aber auch in hohen Konzentrationen lokal in Weichteiltumore appliziert werden. CALCIMYCIN wird bislang nicht systemisch am Menschen eingesetzt (wenngleich Erfahrungen aus Tierversuchen vorliegen), sondern ex vivo zur Behandlung von Oozyten vor in vitro Fertilisationen verwendet.

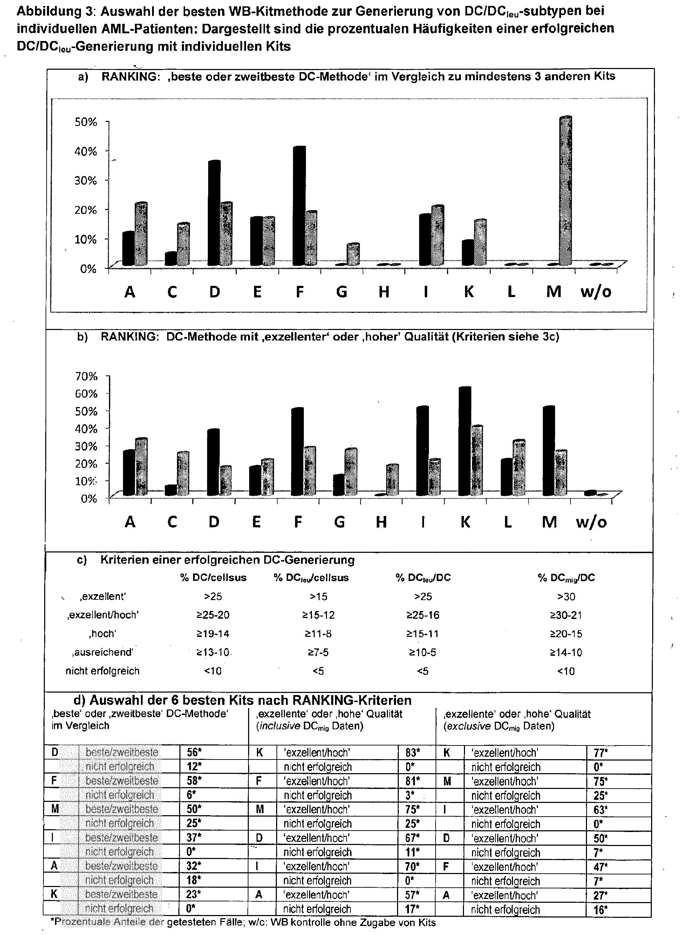

**DC/DCₗₑᵤ-GENERIERUNG EX VIVO- AUSWAHL DER BESTEN KITS (RANKING):** Es wurde gezeigt, dass nicht jedes Kit gleichermaßen gut zur DC(Subtyp)-generierung bei individuellen Patienten geeignet ist (siehe **Abbildung 2a**). Daher wurde von uns eine *"**Rankingliste**"* für Kits zur DC-Generierung erstellt, wobei die prozentual erzielten Werte an DC, an DCₗₑᵤ sowie an reifen migrationsfähigen DC (DC_{mig}) in die Bewertung eingingen: **Abbildung 3a** zeigt die Häufigkeiten, wann einzelne Kits (Zusammensetzung siehe **Tabelle 3**) die BESTE ODER ZWEITBESTE DC-Generierungsmethode waren im Vergleich zu mindestens 3 anderen Kits: Dabei rangierten HS-F, D, M, I, A, K unter den besten oder zweitbesten Methoden, während die verbleibenden Kits schlechter abschnitten (**Abbildung 3a** **und d**). Alternativ sind auch die Häufigkeiten dargestellt, mit denen die DC-Generierung mit Kits als 'EXZELLENT ODER HOCH' (nur orientiert an DC-Subtypqualitäten) eingestuft worden war (**Abbildung 3b****,** Kriterien für die Klassifikation sind in **Abbildung 3c** aufgeführt). Wiederum rangierten die Kits HS-K, F, M, I, D, A auf den Plätzen mit 'EXZELLENT ODER HOCH' DC generierung, wenn Anteile an reifen DC_{mig} in das Ranking mit eingeschlossen (**Abbildung 3d** mittlere Spalte) oder ausgeschlossen wurden (rechte Spalte). Insgesamt ergaben unsere Ergebnisse somit, dass die ersten 6 genannten Kits auf jeden Fall in der Lage sind gute DC/ DCₗₑᵤ-Mengen hervorzubringen, während die verbleibenden Kits bzw Einzelsubstanzen bzw Kontrollen nur selten, wenn überhaupt ausreichende Mengen an DC hervorbrachten.

**INDUKTION DER BLASTENPROLIFERATION EX VIVO:** Es muss ausgeschlossen werden, dass an Patienten verabreichte Kits zu einer ***Proliferation*** (residueller) Blasten (in vivo) führen. Daher wurden im WB-Modell Blastenproliferationstests an 51 Proben durchgeführt: durchflußzytometrisch wurde die Expression des Proliferationsmarkers CD71 (Transferrinrezeptor) auf den Blasten vor (Anteile von doppelt CD71+ und Blastenmarker+ in den Blasten: ,CD71+Bla+/Bla+') und nach Einfluß der Kits untersucht (hier wurden die Anteile an DCₗₑᵤ (die ebenfalls Blastenmarker exprimieren nicht hinzugerechnet: CD71+Bla+DC-/Bla+)). Nur Fälle mit einer Expression von mehr als 10% der Proliferationsmarker vor Kultur wurden in die Analysen eingeschlossen. CD71 ist regelmäßig, aber nicht von allen AML-Fällen auf den (unmodulierten) Blasten exprimiert. Bis auf wenige Ausnahmen (ca 14% der Fälle) fand sich keine Zunahme an CD71+, proliferierenden Blasten nach Kiteinfluss. Somit ist - bis auf wenige Fälle - davon auszugehen, dass die Proliferation von Blasten unter dem Einfluss von Kits nicht angeregt wird, jedoch sollte patientenindividuell die Proliferation der Blasten unter dem Einfluß spezifischer, ausgewählter Kits (insbesondere im Hinblick auf eine in vivo Applikation) untersucht werden. In Zukunft sollen noch weitere Proliferationsmessmethoden eingesetzt werden (CFDA-Assay, durchflußzytometrische Messung von intrazellulären Proliferationsmarkern), um bei möglichst allen Patienten vor WB-Kultivierung einen Proliferationsstatus zu erhalten).
Somit stimulieren die verwendeten Kits nur in Einzelfällen (bzw. nur unter Verwendung einzelner Kits bei individuellen Patienten) die Blastenproliferation im WB-system. Daher empfiehlt sich, den Einfluss *verschiedener* Kits auf Blasten in WB-Proben bei allen individuellen Leukämiepatienten auszutesten und *den* Kit für in vivo Applikationen auszuwählen, der keine Blastenproliferation induziert und gleichzeitig viele DCₗₑᵤ-Anteile hervorbringt.

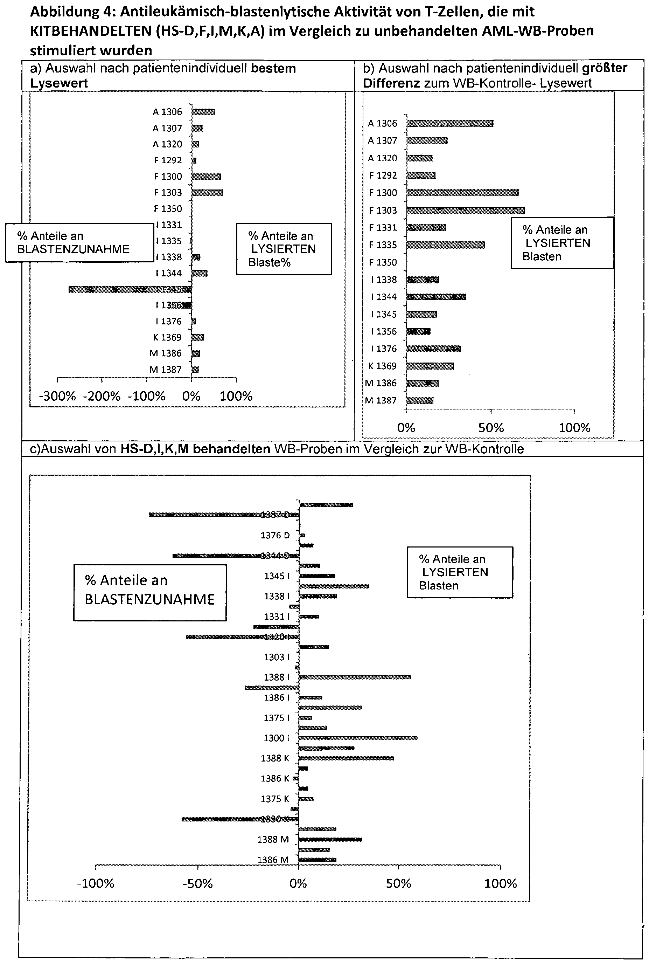

**BEREITSSTELLUNG ANTILEUKÄMISCH WIRKSAMER T-ZELLEN NACH KIT-STIMULATION:** Letztendlich ist ***die antileukämische Funktion der T-Zellen*** im Körper für die Erhaltung einer langanhaltenden Remission entscheidend. Daher wäre erfindungsgemäß eine Optimierung der antileukämischen T-Zell-Funktion sowie die Generierung/Reaktivierung von T-Gedächtniszellen von großem klinischen Nutzen zur Bekämpfung (residueller) leukämischer Zellen im Körper der Patienten. Erfindungsgemäß wurde daher geprüft, ob die Kitbehandlung von WB-Blasten zu einer besseren und effizienteren antileukämischen Aktivität von T-Zellen führt. In **Abbildung 1b** ist schematisch zusammengestellt, wie der ex vivo Nachweis der T-Zellfunktion vollzogen wird: zunächst werden leukämische Blasten mit einem Kit zu DCₗₑᵤ konvertiert (**b1**). Zu den DC-haltigen (=Kitmodulierten Blasten) sowie als Kontrolle Blasten ohne Kitbehandlung in WB-kulturen werden schließlich isolierte T-Zellen aus dem Patienten gegeben und diese in gemischten Lymphozytenkulturen für 7-10 Tage kultiviert (**b2**). Schließlich wird die antileukämische Funktion der so erhaltenen Zellen durch Zugabe von (markierten) leukämischen Zellen in einem Lysetest (durchflusszytometrisch) überprüft: nach Inkubation dieser Zielzellen mit den T-Effektorzellen kann die Abnahme sowie ggf die Zunahme von Blasten quantifiziert werden **(b3).** Dadurch sollte erarbeitet werden, ob die Kitbehandlung von WB-Blasten zu einer besseren und effizienteren antileukämischen Aktivität von T-Zellen führt. **Abbildung 4** zeigt Ergebnisse, die mit KITBEHANDELTEN "modulierten WB-Blasten" im Vergleich zu WB-Kontrollen erzielt wurden: Aufgrund der mit Rankingkriterien ermittelten besten Kits sowie der Tatsache, dass die T-Zellen für die Durchführung von Versuchen begrenzt sind wurden funktionelle Tests mit den Kits HS-A,F,D,I,K,M durchgeführt. Es ist zu sehen, dass nicht immer durch Kitbehandlung von WB antileukämisch wirksame T-Zellen bereitgestellt werden (es können auch T-Zellen entstehen, die nicht blastenlytisch sind), dass die lytische Effizienz der resultierenden T-Zellen variabel ist bzw patientenindividuell beste Kits ausgewählt werden können (**Abbildung 4a**). Auch nur WB ("blastenstimulierte") T-Zellen sind durchaus in der Lage antileukämische stimulierte und zytotoxische T-Zellen (ex vivo) hervorzubringen, wenngleich weniger häufig und weniger effizient. **Abbildung 4b** zeigt, dass patientenindividuell Kits ausgewählt werden können, die die antileukämische Funktion der T-Zellen im Vergleich zur WB-Kontrolle am meisten verbessern. Bei fast allen Fällen konnte (mindestens) ein Kit gefunden werden, der zu einer Verbesserung der antileukämischen T-Zellfunktion führte: Wählte man den besten erzielten LYSEwert nach Kiteinfluß aus und verglich ihn mit der WB-Kontrolle ohne Zusatz von Kits, so konnte bis auf wenige Fälle immer ein Kit gefunden werden, bei dem antileukämische Funktion und *gleichzeitig* eine Verbesserung gegenüber der Kontrolle (ohne Kitzugabe) gezeigt werden konnte. In **Abbildung 4c** sind (parallel erzielte) Ergebnisse mit HS-D, I, K, M-behandelten Kits dargestellt. Diese 4 Kits wurden präferenziell ausgewählt und die Kits HS-A und F zurückgestellt, da die systemische Applikation von TNFa bzw CALCIMYCIN haltigen Kits an Patienten derzeit nicht zugelassen ist. Es ist zu sehen, dass Kit-behandelte WB-Proben Unterschiede aufweisen in der Bereitstellung effizienter antileukämischer T-Zellen. Daher wurde von uns ein ***Ranking*** der antileukämischen Funktion durchgeführt. Dieses ergab, dass die Kits HS-I, K, F, M, gefolgt von HS- A, D am effektivsten im Vergleich zur Vollblutkontrolle antileukämisch ex vivo wirksam waren (Daten nicht gezeigt). Neben den aufgeführten Ergebnissen wurden von uns auch Vergleiche der antileukämischen Wirksamkeit WB- im Vergleich zu Kit-stimulierter WB Proben oder auch unstimulierten TZellen durchgeführt. Auch diese Untersuchungen ergaben, dass nicht alle Kits gleichermaßen und patientenindividuell variabel antileukämisch (besser) wirksame T-Zellen hervorbringen, so dass grundsätzlich detaillierte funktionelle Analysen im Vergleich zu verschiedenen Kontrollen nötig sind, um den patientenindividuell besten Kit herauszufinden, der die beste antileukämische TZellfunktion ergibt.
Begleitend werden ***T-Zell(funktions)profile*** sowie ***Zytokinprofile*** vor/nach Stimulation von T-Zellen mit Vollblutproben (mit oder ohne Kitbehandlung) vergleichend untersucht, um zu erarbeiten, ob regulatorische bzw antileukämisch 'effektive' T-Zellen sowie Gedächtniszellen bzw inhibitorische/aktivierende Zytokinprofile generiert werden. Dazu werden durchflussytometrische Analysen zur Ermittlung der zellulären Profile sowie Analysen zur Ermittlung von Zytokinprofilen sowie Funktionsanalysen durchgeführt.

Somit sprechen die dargestellten Ergebnisse dafür, dass die Immunmodulation von Blasten mit den getesteten Substanzgemischen grundsätzlich möglich ist und regelmäßig gelingt, wenngleich patientenindividuelle Unterschiede auftreten. Diese Kits können AML-Blastenhaltige WB-Proben so modulieren, dass gute antileukämische Wirksamkeit der damit stimulierten T-Zellen resultiert, wenngleich dies wiederum nicht für jedes Kit bzw. jede individuelle Patientenprobe gleichermaßen gut gelingt. Letztendlich kann aber die beste Kombination an Kits in WB ermittelt und schließlich auf ihre funktionell ausgetestet werden, um das patientenindividuell beste Kit für eine in vivo Applikation auszuwählen.

**KIT-AUSWAHL: Tabelle 3** zeigt die erfindungsgemäß erarbeiteten Kits sowie die wichtigsten Erkenntnisse und klinischen Erwägungen vor einem Einsatz am Patienten (Details dazu siehe weiter unten): Der patientenindividuell beste aus den ersten 4 vorrangig ausgewählten Kits (HS-D, I, K, M) soll dabei ermittelt und schließlich dem Patienten verabreicht werden. HS- C, F und A werden zunächst als nachrangige Kits vorgestellt. Grund dafür ist, dass CALCIMYCIN bislang nicht, sowie TNFa derzeit nicht mehr systemisch eingesetzt werden darf und TNFa in einer Dreifachkombination verabreicht zurückgestellt werden soll. Die Kombination HS-E sowie die Einzelsubstanzen HS-G, H und L wurden mitgetestet, sind grundsätzlich für den Einsatz am Patienten möglich, aber im Vergleich zu den anderen Kombinationspräparaten nicht so effizient in der Bereitstellung von DC(Subtypen); HS-E, G, H und L sollen daher ebenfalls nachrangig aufgeführt werden. Die hier vorrangige erfindungsgemäß vorgestellte Kitauswahl ist daher: HS-D, HS-I, HS-K, HS-M. Konzeptionell steht hinter der Auswahl dieser Kits, dass sie verschiedene immunmodulatorische/-stimulierende Wirkungen entfalten sollen (**Tabelle 1, 2**): in allen Kombinations-Kits ist GM-CSF enthalten, ein Zytokin, das die myeloische Zellproliferation und - Differenzierung induziert und positiv auf die Bereitstellung und Regeneration von Stammzellen, immunkompetenten Zellen, Granulozyten sowie auch von DC im allgemeinen wirkt ohne die Blastenproliferation zu begünstigen (**Tabelle 3**).
Im Falle von Kit **HS-I** (GM-CSF und PICIBANIL) soll durch die zusätzliche Applikation des Streptokoccenlysats eine Aktivierung immunreaktiver Zellen erfolgen sowie die Konversion der myeloischen Blasten zu DCₗₑᵤ begünstigt werden, wie dies für die ex vivo Generierung von DC bereits bekannt ist. Möglicherweise könnten die so behandelten AML-Patienten auch vom Einfluß von PICIBANIL auf das Gefäßsystem profitieren (antiangiogenetische Wirkung, erhöhte Durchlässigkeit des Endothels für immunreaktive Zellen oder humorale Faktoren).
Im Falle von **HS-M** (GM-CSF und PGE₁) soll PGE₁ zusätzlich zu GM-CSF verabreicht werden. In Analogie zu PGE₂ konnte gezeigt werden, dass PGE₁ auch die Differenzierung von DC begünstigt; PGE₁ wird (in höheren Konzentrationen) auch für die Behandlung der VOD Erkrankung von Patienten nach SCT eingesetzt. Somit könnte HS-M gerade für Patienten nach SZT einen gewünschten zusätzlichen vasodilatatorischen Effekt bewirken.
**HS-K** (GM-CSF und PGE₂) und **HS-D** (GM-CSF und PGE₂ und PICIBANIL) sind Kits, die PGE₂ enthalten. PGE₂ erhöht den Reifegrad bzw die Migrationsfähigkeit der DC. HS-D enthält zusätzlich noch PICIBANIL, das möglicherweise die immunmodulierende Funktion des Kits (gefolgt durch eine verbesserte T-Zellwirkung) noch steigert.
Nachrangige Kits: TNFα haltige Kits **HS-A** (GM-CSF und TNFα) sowie **HS-C** (GM-CSF und TNFa und PGE₂) enthalten TNFα in Zwei-bzw Dreilachkombinationen: TNFα ist als Akutphasenprotein bei entzündlichen Prozessen, als Regulatorzytokin zellulärer Prozesse sowie zur Aktivierung des Immunsystems -beispielsweise als Induktor der DC Reifung - beteiligt. TNFα wurde bislang lokal zur Behandlung von Weichteiltumoren (in hoher Konzentration) eingesetzt sowie systemisch (in niedriger Konzentration) zur Behandlung von Patienten mit fortgeschrittenen Neoplasien. Zunächst soll vorrangig HS-A bei Leukämiepatienten (in niedrigen TNFα Dosen) eingesetzt werden, sobald Erfahrungen mit den erstrangig genannten Kits in der Patientenbehandlung vorliegen auch HS-C. Weiterhin genannt sei auch HS-F (GM-CSF und CALCIMYCIN): CALCIMYCIN hat antibiotische Wirkung sowie Wirkung auf den Calciumionenaustausch, wirkt als Entkoppler und ATPase Inhibitor. Im Tierversuch wurde gezeigt, dass (i.v. verabreicht) es -in extrem hoher Dosierung- Entzündungen bzw Anaphylaxien auslöst. CALCIMYCIN wurde beim Menschen extrakorporal zur Befruchtung von Oozyten eingesetzt, bislang aber nicht systemisch am Menschen appliziert. Geplant ist, HS-F bei Leukämiepatienten (in niedrigen CALCIMYCIN Dosen) einzusetzen, sobald. Erfahrungen mit den erstrangig genannten Kits in der Patientenbehandlung vorliegen. Im Falle von Kit **HS-E** (GM-CSF und INTRON) könnte durch zusätzliche Applikation des Zytokins IFNα2b (=INTRON) zusätzlich eine Aktivierung des Immunsystems erfolgen sowie die Konversion der myeloischen Blasten zu DCₗₑᵤ begünstigt werden, wie dies von der chronisch myeloischen Leukämie bekannt ist, jedoch war HS-E ebenso wie die getesteten Einzelsubstanzen **HS-G** (GM-CSF), **HS-H** (IFNα2b) und **HS-L** (PICIBANIL) zwar grundsätzlich für den Einsatz am Patienten möglich, aber im Vergleich zu den Kombinationspräparaten nicht so effizient in der Bereitstellung von DC(Subtypen); HS-E, G , H und L sollen daher nachrangig aufgeführt werden.
Im letzten Schritt müssen KORRELATIONSANALYSEN durchgeführt werden, um die Rolle bzw Menge an Subpopulationen an DC, T-Zellen sowie an Zytokinkonzentrationen im Kontext der antileukämisch lytischen Funktion zu untersuchen. Die Daten dafür stehen derzeit noch nicht zur Auswertung zur Verfügung.

**FAZIT UND UMSETZUNG DER ERFINDUNG IN DIE KLINIK:** Auch erfolgreich chemotherapierte oder stammzelltransplantierte AML-Patienten rezidivieren häufig, da leukämische Zellen im Körper verbleiben und schließlich für den Rückfall verantwortlich sind. Die hier dargestellte Erfindung adressiert diese residuellen Blasten: sie sollen mit Hilfe von individuell ausgewählten und den Patienten applizierten Kits (bestehend aus klinisch zugelassenen Substanzen, Wachstumsfaktoren und Immunmodulatoren), so zu DCₗₑᵤ moduliert werden, dass sie als eine leukämieantigenpräsentierende 'DC-Vakzine' antileukämische T-Zellen aktivieren, ein immunologisches T-Zell-Gedächtnis anlegen, zusätzlich auch andere zelluläre und humorale immunologische Barrieren überwinden helfen und somit die Patienten vor Rezidiven schützen.

Es konnte grundsätzlich gezeigt werden, dass aus VOLLBLUT (WB) von Patienten mit myeloischer Leukämie (AML) mit "Standard-Verfahren" sowie minimalisierten Kombinationen aus Immunmodulatoren (Kits) DC/DCₗₑᵤ generiert werden können und diese antileukämische T-Zellen nach Stimulation hervorbringen können ohne dass die Blastenproliferation induziert wird. Dabei waren patientenindividuelle Unterschiede zu erkennen: nicht bei jedem Patienten war jeder Kit erfolgreich und nicht bei jedem Patienten gelang die antileukämische T-Zellaktivierung gleichermaßen gut. Somit sollte - im klinischen Kontext betrachtet- möglich sein, nach Vortestungen in WB-Proben der Patienten durch Applikation von (patientenindividuell ausgewählten) optimalen Kombinationen an Immunmodulatoren die in vivo Produktion von 'DC/DCₗₑᵤ' zu induzieren, die in vivo antileukämische T-Zellen induzieren und somit die Aufrechterhaltung von Remissionen ermöglichen. Diese AUSTESTUNG sollte in der Zellkultur mit blastenreichen WB Proben (entnommen in in akuten, blastenreichen Krankheitsstadien), die BEHANDLUNG der Patienten sollte in Stadien erfolgen, in denen die Patienten möglichst nicht zu sehr (z.B. krankheits- oder chemotherapiebedingt) immunkomprimiert sind, um eine Regeneration immunkompetenter Zellen zu gewährleisten. Die vorgestellte Erfindung ist potentiell zur Behandlung von allen Patienten jeden Alters mit myeloischen Leukämien (chronischen oder akuten myeloischen Leukämien oder Myelodysplasien) vor oder nach SCT möglich. Der Ansatz ist nicht HLA-restringiert und ohne Kenntnis beteiligter leukämischer Antigene möglich. Grundsätzlich können Patienten in akuten sowie in chronischen Krankheitsstadien/Remissionen mit dem vorgestellten Konzept therapiert werden. Die klinische Wirksamkeit, Verträglichkeit und Sicherheit der individuell ausgewählten (sehr niedrig dosierten) Kits soll zunächst an ca 10-15 Patienten im Rezidiv nach SZT geprüft werden
Ex vivo wurde von uns gezeigt, dass unterschiedliche Kits bei individuellen Patientenproben unterschiedlich wirken bzw. unterschiedlich effizient sind, so dass ein personalisiertes Behandlungsprotokoll abgeleitet werden soll. Bei jedem Patienten -zunächst mit AML im Rezidiv - sollen daher folgende Kits an WB-Proben getestet werden (**HS-I, K, M und evtl D**) und der effizienteste Kit ex vivo ausgewählt werden: ein Kit, der die beste Blastenmodulation erzielt (beste DC/DCₗₑᵤ-subtypen) ohne eine Blastenproliferation zu induzieren, der die effektivste antileukämische T-Zellantwort mit Patienten-T-Zellen induziert (ohne überschießende T-Zellreaktionen, beispielsweise im Rahmen einer GvH hervorzubringen) und das beste Mikromilieu/Zytokinprofil hervorbringt. Durch Behandlung des Patienten mit diesem Kit könnte somit eine Überwindung von immunologischen Barrieren erzielt und eine funktionierende zelluläre und humorale Immunität im Patienten wiederhergestellt werden.
Vorrangig sollen 3-4 Kits an ausgewählten rezidivierten Patienten nach SZT zum Einsatz kommen, deren Einzelsubstanzen gut verträglich sind bzw sogar klinisch/therapeutisch bekannte Komplikationen bei dem genannten Patientenkollektiv minimieren können.
Grundsätzlich könnten auch Kits mit potenziell risikoreicheren Komponenten (z.B TNFa bzw CALCIMYCIN) zum Einsatz kommen, wenngleich schwere Nebenwirkungen wegen der hier sehr niedrigen Dosierung nicht zu erwarten sind. Grundsätzlich wäre auch die Applikation von Einzelsubstanzen (GM-CSF, INTRON, PICIBANIL) möglich, aber möglicherweise nicht so effektiv in der Bereitstellung antileukämischer T-Zellen.
Die ***Applikationsform*** (intramuskulär (i.m.), subkutan (s.c.), intravenös (i.v.), intradermal (i.d.)) soll nach Vorschriften und Empfehlungen des Arzneimittelherstellers erfolgen (**Tabelle 3,** Spalte 4), wobei grundsätzlich -sofern möglich- i.v. Applikationen bevorzugt werden, um einen möglichst direkten Einfluß der Substanzen auf leukämische Zellen im Blut zu gewährleisten.
In einigen Zellkultur-Vorversuchen konnte gezeigt werden, dass die *sequenzielle* im Vergleich zur *gleichzeitigen* Zugabe der Substanzen zu den WB-Kulturen vergleichbare Mengen an DC-subtypen hervorbringt (Daten nicht gezeigt). Dabei muss auch geklärt werden, ob GM-CSF iv als Dauerinfusion verabreicht werden soll oder in ebenso vom Hersteller empfohlenen Frequenzen.
Die ***Dauer*** der Anwendung am Patienten kann von mindestens 3 Monaten bis zu 2 Jahre dauern und muss ebenfalls im Kontext der klinischen Anwendung am Patienten entschieden werden: Die Hersteller der hier aufgeführten Substanzen empfehlen eine Behandlungsdauer von bis zu 180 Tagen (**Tabelle 3**, Spalte 7). Voraussichtlich wird die Anwendung auf 3 bis 4 Monate beschränkt sein, falls Patienten im Rezidiv nach SZT behandelt werden. Vorteil der Anwendung der Kits an dieser Patientengruppe ist, dass die Wirkungen auf (zahlreich im Blut vorhandene) Blasten direkt untersucht werden können. Falls diese Behandlungen begleitend zu DLI Behandlungen erfolgen sollten wäre ein weiterer Vorteil die Verabreichung definierter Mengen an T-Zellen und somit der hauptsächlich adressierten Effektorzellen. Bei Behandlungen von Patienten in Remission der Erkrankung im Zuge einer Erhaltungstherapie sollte die Behandlung (in Analogie zu anderern Erhaltungstherapien) über bis zu 2 Jahre erfolgen. Je nach Ergebnissen eines Monitorings (s.u.) könnte eine Verlängerung bzw Verkürzung der empfohlenen Behandlunsgzeit resultieren.
Die *Dosierung* der Substanzen soll mit sehr niedrigen, immunmodulierenden Dosen erfolgen und orientiert sich an den verwendeten Konzentrationen im WB-Kulturansatz, sie könnte grundsätzlich auch auf höhere Dosen, wie vom Hersteller empfohlen, gesteigert werden (**Tabelle 3,** Spalte 4 und 5). Wahrscheinlich wird die optimale Dosierung nach Vorversuchen im Tiermodell erarbeitet werden müssen: Zunächst ist geplant die ausgewählten Kits in einigen Tier-Vorversuchen an immunkomprimierte Ratten oder Mäuse (z.B. NOD-SCID) zu verabreichen (denen menschliche AML-Blasten und T-zellen appliziert wurden): die 2-3 besten Kits sollen dabei in verschiedenen Dosierungen parallel (im Vergleich zu Blutproben ohne Kitbehandlung) in vivo an 2-3 immunkomprimierten Mäuse/Rattengruppen untersucht werden, um zu klären, ob es zu einer Blastenreduktion, zu einer antileukämischen T-Zellaktivierung, zur Bereitstellung von T-zellsubtypen (z.B Effektorzellen, Gedächtniszellen) sowie aktivierenden Zytokinmilieus kommt.
**Tabelle 3** zeigt die erfindungsgemäß hier vorgestellten Kits und gibt einen Überblick über deren Zusammensetzung, über ex vivo verwendete Konzentrationen an Einzelsubstanzen sowie klinisch relevante Überlegungen zur Dosierung, Applikationsform und Dauer der Behandlung mit diesen. Die vorrangige erfindungsgemäß vorgestellte Kitauswahl ist: HS-D, HS-M, HS-K, HS-I, Ferner sind einige Daten über die DC-Generierungseffizienz bzw antileukämische Aktivität Kitbehandelter T-Zellen dargestellt. Auch hier zeigt sich, dass die genannten Kits gute DC-Generierungs- bzw antileukämische Aktivität stimulierter T-zellen aufweisen.
Insgesamt sei erwähnt, dass derzeit noch kein konkretes Behandlungsprotokoll am Menschen verfügbar ist. Daher sind im Text bzw. in den Abbildungen angegebene Dosierungen, Applikationsvorschläge, etc. nur orientierend gedacht.
Neben Untersuchungen der WB-Proben vor/nach ihrer ex vivo Verarbeitung müssen regelmäßige Untersuchungen von Blutproben der Patienten während der in vivo Kitbehandlung durchgeführt werden (*Monitoring*), um deren Wirkungs- und Nebenwirkungsprofile zu erfassen. Diese Untersuchungen müssen VOR Beginn der Applikation (Kontrollwerte) sowie zu mehreren Zeitpunkten im VERLAUF der Behandlung durchgeführt werden. Diese Zeitpunkte sollten sich nach den Zeitpunkten richten, die bei anderen immuntherapeutischen Behandlungen empfohlen sind: Im Vordergrund steht zunächst die Quantifizierung leukämischer Zellen mit Hilfe durchflusszytometrischer Verfahren (Blastenprofil) sowie mit molekularen Profilen (Überexpression leukämieassoziierter Antigene, PCR); ggf auch DC/DCₗₑᵤ, sofern sie im Blut nachweisbar sind. Daneben muss eine Charakterisierung der Rolle an der Leukämieabwehr beteiligter zellulärer und löslicher Faktoren erfolgen: T-Zellsubpopulationen (regulatorisch, CD4/CD8, Gedächtnis/Effektor-T-Zellen, T-Zellen mit definiertem Vβ-Profil, funktionale Eigenschaften beteiligter T-Zellen (antileukämische Wirksamkeit), ggf. Vorliegen von LAA-spezifischen T-Zellen (Tetrameranalysen)) humorale Faktoren (inhibitorische (z.B IL-10,TGFβ, CXCR4)/stimulierende lösliche Faktoren, Chemokine, Zytokine (z.B. IL-2, IL-12, IL-17, IFNγ), gemessen in Zytokinenachweis-Analysen).
Durch *Monitoring* von löslichen und zellulären Faktoren zu verschiedenen Zeitpunkten im Krankheitsverlauf und durch Korrelationen der Daten mit klinischen Ereignissen (z.B. Rezidiv) kann schließlich die klinische Relevanz, der Behandlungserfolg und die prognostische Wertigkeit unseres Ansatzes erarbeitet werden.
Es ist im Rahmen der vorliegenden Offenbarung, dass Ausführungen zu den einzelnen Aspekten der Erfindung, wie sie Gegenstand der beigefügten unabhängigen Ansprüche sind, soweit sie spezifisch im Zusammenhang mit einem der Aspekte gemacht sind, auch Ausführungen zu allen anderen Aspekten der Erfindung darstellen. Insoweit sind Ausführungsformen eines Aspektes der Erfindung auch Ausführungsformen der anderen Aspekte der Erfindung.

### Versuchsergebnisse aus dem Einsatz von Kits an leukämiekranken Ratten:

Zwischenzeitlich wurden die Kits in einem Leukämie-Rattenmodell geprüft, was als Beispiel eines Belegs der Wirksamkeit der Kits auch in vivo aufgeführt werden soll:
**BN-(,brown norway) Ratten** sowie **PVG.1N Ratten** sind Ratten mit demselben MHC Profil. Bei BN-Ratten kann durch Injektion von BNML-leukämischen Zellen eine AML-M3 erzeugt werden. Die Leukämie manifestiert sich innerhalb von 14-17 Tagen zunächst in der Milz, später dann auch im peripheren Blut und führt dann innerhalb von 7 Tagen zum Tode. Aus der Schwanzvene können Blutproben für Zellanalysen durchgeführt werden; nach der Opferung der Tiere (nach 23 Tagen, kurz vor ihrem AML-bedingten Tod) können darüberhinaus Zellanalysen auch an Milz- sowie an Blutzellen nach Herzpunktionen durchgeführt werden.

**EX VIVO DC/DCₗₑᵤ GENERIERUNG AUS WB LEUKÄMIEKRANKR RATTEN:** In Analogie zu menschlichem blastenhaltigen Blut führten wir Kultivierungen von AML-Ratten-WB mit den Kits HS-D, I, K, M im Vergleich zu WB Kontrollen durch (Es wurden dieselben Substanzen wie beim Menschen verwendet; Ausnahme: *Ratten*-GM-CSF). Erste Auswertungen ergaben, dass aus Rattenblut von 5 Tieren ebenso wie aus menschlichem leukämiekranken WB DCₗₑᵤ generiert werden können (durchschnittlich 10-35% innerhalb der gesamten Zellfraktion mit einer Konvertierbarkeit der Blasten zu DCₗₑᵤ von 30-50%). Am erfolgreichsten war HS-I, gefolgt von HS-M und HS-K. Eine Induktion der Blastenproliferation (Koepression von Ki67) war nach Behandlung mit keinem der Kits zu sehen. Darüberhinaus konnte nach Kokultur von DCₗₑᵤ-haltigen, Kit-behandelten WB-Proben mit autologen Ratten Tzellen eine erhöhte Blastenlyseaktivität gegen BNML-Blasten bei allen Kits nachgewiesen werden. Die höchste antileukämische Wirksamkeit wiesen HS-I- WB-stimulierte Tzellen auf. Genauere Analysen stehen noch aus. Da grundsätzlich Blasten in Rattenblut zu DCₗₑᵤ konvertierbar waren sollten nun in vivo Behandlungen durchgeführt werden.

**IN VIVO BEHANDLUNG VON PVG.1N RATTEN MIT PICIBANIL, PGE1 und PGE2 (SAFETY-DATEN):** Safety Daten waren an insgesamt 4 Ratten des gesunden Stamms PVG.1N durchgeführt worden: ihnen wurden die klinisch intendierten Endkonzentrationen der Einzelsubstanzen iv in folgenden Dosen verabreicht: Picibanil (0.35 µg/Ratte), PGE2 (1 µg/Ratte), PGE1 (1 µg/Ratte) und Placebo (PBS). Die Ratten zeigten keinerlei (schwere) Nebenwirkungen ((S)AE): Atmung, Schlaf, Nahrunsgaufnahme, Mobilität, Haut, Gewicht waren komplett normal bzw wie bei den Kontrolltieren (Daten nicht gezeigt). Durchflusszytometrische Analysen an Blutzellen der PVG.1N Ratten wiesen keinerlei Unterschiede im Vergleich zu den Placebokontrollen auf (Teff-, Treg-, CD4-, CD8-, NK-, NKTZellen, Daten nicht gezeigt).

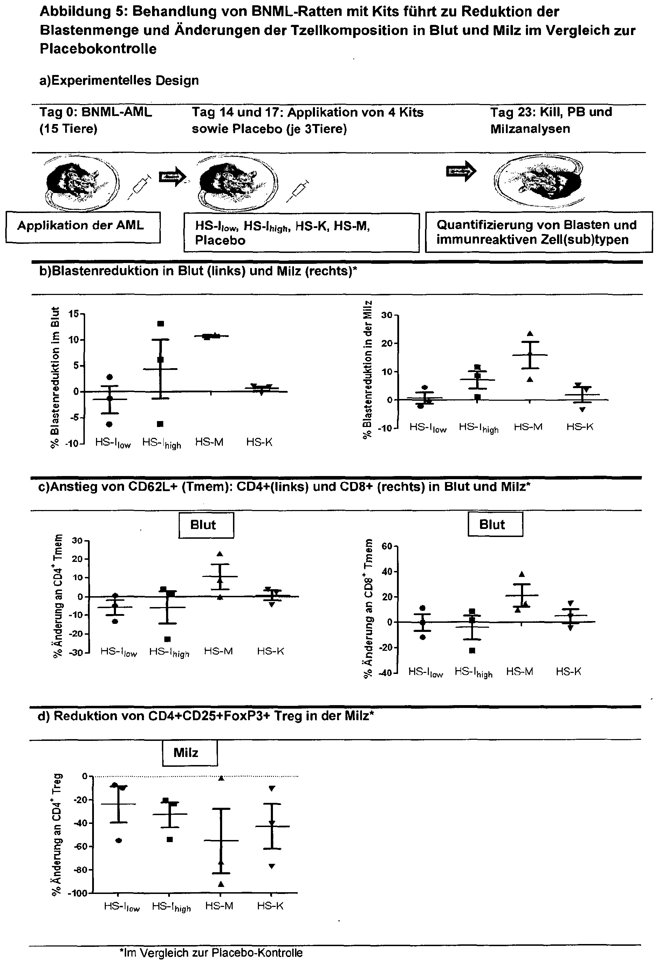

**IN VIVO BEHANDLUNG VON LEUKÄMIEKRANKEN BN RATTEN MIT KITS (EFFICACY-DATEN):** Da die Verträglichkeit der von uns verwendeten Substanzen sehr gut ist wurden nun BN-Ratten nach Induktion der Leukämie (Tag 0) am Tag 14 und 17 mit 4 Kits im Vergleich zur Placebokontrolle behandelt (i.v Applikation in Penisvene, **siehe** **Abbildung 5** **a**): je 3 Ratten erhielten:
HS-I_{low}: GM-CSF (1µg/Ratte) + Picibanil (0.175 µg/ Ratte, entsprechend 0.5 KE im Menschen)
HS-I_{high}: GM-CSF (1µg/ Ratte) + Picibanil (0.35 µg/ Ratte, entsprechend 1 KE im Menschen)
HS-K: GM-CSF (1µg/ Ratte) + PGE₂ (1µg/ Ratte)
HS-M: GM-CSF (1µg/ Ratte) + PGE₁ (1µg/ Ratte)
Placebo: PBS

Die Wirksamkeit (Efficacy) der Kits wurde am Tag 23 als Vergleich der **Blasten**mengen in Blut und Milz nach Kit-behandlung im Vergleich zur Placebokontrolle ermittelt: Während HS-I_{low} (mit niedrig dosiertem Picibanil) sowie HS-K (PGE2 haltig) keine Unterschiede in den Blastenmengen im Vergleich zur Kontrolle aufwiesen waren die Blastenanteile nach Behandlung mit HS-I_{high} um 4.4-6.3% in Blut bzw Milz reduziert, nach HS-M Behandlung waren die Blastenanteile sogar um 10.7-15.8% significant reduziert. Analysen der immunreaktiven Zellen im Blut zum Zeitpunkt der Opferung der Ratten ergab folgendes Bild: **CD62L+CD4+ bw CD8+** T(memory)zellen waren nach Behandlung mit HS-M (nicht aber nach Behandlung mit den verbleibenden Kits) in Ratten um 10,4% (CD4+) bzw 21,1% (CD8+) im Blut erhöht nachweisbar **(****Abbildung 5c****).** Auffallend war auch die prozentuale Reduktion an **CD4+FoxP3+CD25+** regulatorischen Tzellen nach Behandlung der Ratten mit allen Kits (um -24,3% (HS-I_{low}) bis zu -55,6% (HS-M)) in der Milz im Vergleich zur Kontrolle **(****Abbildung 5d****).** Weitere Daten über immunreaktive Zellen stehen derzeit noch nicht zur Verfügung.
Zusammenfassend zeigen unsere mit Rattenblut erzeugten Ergebnisse, dass die ex vivo Generierung von DC/DCₗₑᵤ ähnlich gut wie mit menschlichem WB gelingt ohne die Blastenproliferation zu steigern. Die Behandlung von gesunden Ratten mit den Einzelsubstanzen zeigten eine sehr gute Verträglichkeit der Substanzen; die Behandlung AML-M3-leukamiekranker BN-Ratten mit unterschiedlichen Kits HS-K,M,I, zeigten, dass in nur 10 Tagen einer Behandlung mit unserem immunmodulatorischen Behandlungskonzept eine Blastenreduktion um bis zu 15.8 % erreicht werden konnte, jedoch nicht jeder Kit gleichermaßen gut bei dieser Leukämieform wirkt.

**FAZIT:** Basierend auf der vorliegenden Erfindung wird es zeitnah möglich sein, ein "in vivo Protokoll" zu erarbeiten, das die Applikation minimalisierter Kombinationen aus Immunmodulatoren (ohne Reinraum ('GMP-Facility')-Bedarf für die Herstellung) bei Patienten mit myeloischen Leukämien ermöglicht. Das detaillierte Design eines personalisierten Behandlungsprotokolls für Patienten mit myeloischen Leukämien mit unterschiedlichen bzw unterschiedlich kombinierten immunmodulatorisch wirksamen Einzelsubstanzen sowie das detaillierte Konzept eines Immunmonitorings muss nach Rücksprache mit behandelnden Ärzten vor dem Start der Behandlungen erfolgen und wird sich an anderen immuntherapeutischen Behandlungsprotokollen orientieren. Expertise für immuntherapeutische Behandlungen sind an den für die Behandlung der Patienten vorgesehenen Institutionen gegeben. Ein routinemäßig erhobenes diagnostisches Begleitprogramm zur Untersuchung der Blutproben sowie klinische Befunde über Patientenverläufe stehen zur Verfügung.
Somit können für die klinische Umsetzung der Erfindung vorhandene Erfahrungen, Expertisen und Synergismen genutzt werden, um -zunächst- AML-Patienten im Rezidiv nach allogener SZT zu behandeln.

**Tabelle 1: Generierung von DC/DCIₑᵤ aus AML-blasten-haltigen Mononukleären-(MNC) oder Vollblut (WB)-Zellen mit verschiedenen DC-Differenzlerungsmethoden bzw bestimmten Kits**

| **a)DC-Differenzlerungsmethoden bzw Kits zur Generierung von DC/DCₗₑᵤ aus blastenhaltigen MNC (a1-a4) oder WB (a1-a5)** | | | | | |
|---|---|---|---|---|---|
| DC-Generierungs methode | **'CA' -a1-** | **'MCM' -a2-** | **'PICI' -a3-** | **'INTR' -a4-** | **'Kits': HS-A, C, D, E, F, G, H, I, K, L, M' -a5-** |
| Kulturzeit | 3-4 Tage | 10-14 Tage | 9-11 Tage | 10-14 Tage | 8-10 Tage |
| DC-Generierung s-Methoden | IL-4, CALCIMYCIN | GM-CSF, IL-4, TNFα, FL, IL-1β, IL-6, PGE₂, | GM-CSF, IL-4, PGE₂, TNFα, PICIBANIL | GM-CSF, INTRON, TNFα | Diverse Kombinationen aus 1-3 klinisch zugelassenen Einzelsubstanzen/Immunmodulatoren |
| Wirkmechani s-mus | Umgehung der Zytokin induzierten DC-Differenzie rung | Zytokin induzierte DC-Differenzie rung, PGE₂ steigert CCR7-Expression und verbessert die Miqration | Bacterielles Lysat und PGE₂ stimulieren die DC Differenzie rung | Zytokin induzierte DC-Differenzierung, | DC-Differenzierung durch Kombination aus Immunmodulatoren/ Dangerfaktoren/Zytokinen/ Reifungsinduktoren |
| IL-4,-1β,-6: interleukin 4, -1β, -6 | | PGE₂: Prostaglandin E2 | | INTRON: Interferon alpha2b | |
| TNFα: tumor necrosis factor alpha | | GM-CSF: granulocyte macrophage colony stimulating factor | | PICIBANIL: OK432 (Bakterienlysat aus Streptococcus pyogenes | |
| FL FLT3 Ligand | | | | | |
| Details zu Wirkstoffen siehe Tabelle 2 | | | | | |

| **b) Vorteile und Wertigkeit einer DC-Generierung aus Vollblutproben** | | | | | |
|---|---|---|---|---|---|
| **Vorteile** | | | **Kommentare in Bezug auf die klinische Relevanz bzw eine Umsetzung in die klinische Anwendung** | | |
| Simulation eines physiologischen Systems | | | Übertragbarkeit in die Klinik | | |
| Quantifizierbarkeit und Charakterisierbarkeit an Reaktionen | | | Rückschlüsse auf biologische Mechanismen/Reaktionsprofile | | |
| beteiligter Zellen | | | -mögliche Blastenzunahme quantifizierbar | | |
| -Anteile an Blasten | | | -interessante immunreaktive Zellen (z.B regulatorische, | | |
| -Anteile an Tzell(Sub)populationen, NK, NKTzellen | | | Effektor-Memory, Integrin-positive T-Zellen, NK,NKTzellen) | | |
| - Erfolg einer Blastenkonvertierung zu DCₗₑᵤ bzw DCsubtypen gut quantifizierbar | | | -In vivo sind DC im Blut schwer quantifizierbar wegen ihrer Migration ins Gewebe | | |
| Analyse des Microenvironment auf immunstimulierende und -inhibierende Einflüsse möglich | | | Aufdeckung von, Escape- sowie antileukämisch funktionalen Mechanismen und möglichen Zusammenhängen; Entwicklung von entsprechenden therapeutischen Strategien | | |
| Funktionalitätstests im Vollblut möglich | | | Antileukämische Reaktionen quantifizierbar (Abnahme/Zunahme von Blasten), Effektorzellprofile messbar -(Proliferation, Subtypen) | | |

**Tabelle 2: Wirkstoffe in den Kits zur Immunmodulation von AML-Blasten**

| **Wirkstoff** | **Funktion/Wirkung** | **Bisherige Klinische Anwendung** |
|---|---|---|
| **GM-CSF** Leukine Sargramostin) | -induziert Proliferation und Differenzierung hämatopoetischer Zellen (Vorläuferzellen, Neutrophile, Monozyten, Makrophagen, myeloische DC) | (1)AML-Patienten>55 Jahre: nach Chemotherapie zur Verkürzung der Neutrophilen-Recovery, zur Reduktion der Infektinzidenz |
| | | (2)Mobilisierung von Stammzellen ins PB (zur Sammlung von Stammzellen) |
| | | (3)Myeloische Zellrekonstitution nach autologer/allogern SCT bei ALL, NHL, HD |
| | -reduziert Neutropeniedauer | (4) Behandlung von Transplantatversagen nach autologer/allogener SCT |
| | -verringert Infektionsinzidenz Reduziert Gabe antimikrobieller Substanzen | |
| | | (5) Monozyten/DC-aktivierung bei Melanom-Patienten in vivo |
| | | (6) Immunmodulation von Blasten (s.c oder iv) |
| **TNFα** Kachexin | -Zytokin, das Akutphasenproteine aktiviert | (1)Lokale Behandlung von Sarkomen, nicht resezierbaren Tumoren, Melanomen der Extremitäten |
| | -Induktion von Apoptose, Zellproliferation,-Differenzierung, Zytokinausschüttung | (2)Systemische (i.v.) Behandlung von Patienten mit fortgeschrittenen Neoplasien |
| | -Auslösung von Entzündungen, Nekrosen, Kachexie, Fieber Autoimmunreaktionen, | |
| **INTRON** IFNα2b | -Zytokin mit immunmodulierender Wirkung | (1)Behandlung der Hepatitis B und C |
| | | (2)Behandlung der CML |
| | -wichtig in der Abwehr von virusinfizierten Zellen, | (3) Behandlung von Myelomen, |
| | | (4)Behandlung von follikulären Lymphomen |
| | -wichtig bei der Aktivierung des Immunsystems | (5)Behandlung von karzinoiden Tumoren |
| | | (6)Behandlung von Melanomen, |
| | -Regulatoren der Zellproliferation | (7)Behandlung der Haarzellleukämie |
| | | (Verabreichung i.v, i.m., s.c.: an gesunden Probanden liefern i.v und s.c/i.m. Applikationen vergleichbar) |
| **PGE₁** (Alprostadil) | -vasodilatatorische Eigenschaften (durchblutungssteigernd) | (1)Behandlung der Venoocclusiven Erkrankung (VOD) nach SCT |
| | | (2)Behandlung von erektilen Dyfunktionen |
| | | (3)Offenhaltung des Ductus Arteriosus Botalli |
| | -anti-Platelet-activity | (4) Behandlung von Ischämien der Extremitäten (i.v Verabreichung) |
| **PGE₂** (ProstinE2) | -wird von (aktivierten) Blutplättchen, evtl von Erythrocyten gebildet (durch Aktivierung des Ca-transport in Erys): beteiligt an Koagulation! | (1) Weheninduktion, Geburtsförderung, Aborteinleitung |
| | | (2) Applikation iv, sc, im |
| | -kontraktionsfördernd | |
| **PICIBANIL** OK432 (Chugai Pharma) | -Streptococcus pyogenes (niedrig virulent, H₂O₂ u. Penicillinbehandelt) behandelt | (1) intraläsionale Behandlung von benignen Lymphatischen Geschwulsten (Lymphangiome, zytische Hygrome),Head and Neck' Tumoren |
| | -bewirkt Aktivierung von immunreaktiven Zellen, Zytokinfreisetzung, erhöht Durchlässigkeit von Endothelien, antiangiogenetische Wirkung (Hitayama 2013) | (2)intrauterine Behandlung bei zystischen Hygromen |
| | | (3)intratumorale Injektion von unreifen DC und OK432 in Tumornähe bei Pankreas-Ca |
| | | (4)Pleurodesis-Induktion durch pleurale Infusion von OK432 |
| | | (5) Immunmodulation bei Plattenepithelcarcinomen (id oder in die Nähe des Tumors) |
| | | i.m., i.d oder s.c gemäß Zulassung |
| **CALCIMYCIN,** Calciumlonophor, A23187 (Sigma) -Produkt von Streptomyces chartreusensis | -antibiotische Wirksamkeit gegen gram positive Bakterien | (1)Ex vivo: Behandlung von Oozyten vor der in vitro Fertisisation |
| | | (2) Schwein: i.v. Gabe (hochkonzentriert 5mg/kg) indiziert anaphylaktischen Schock |
| | -erhöht Ca+2 ionen intrazellulär | |
| | -Entkoppler oxidativer Phosphorilierung | (3) Ratte: intrapleurale Injektion erzeugt Rippenfellentzündung |
| | | (4) Meerschweinchen: Inhalation erzeugt Lungenentzündung |
| | -Inhibitor der mitochondrialen ATPase | |

**Tabelle 3: Zusammensetzung der getesteten Kits**

| **Kits** | **Kommentare zu 1,2,3,** | | | | | | | |
|---|---|---|---|---|---|---|---|---|
| | **Dosierung in ZELLKULTUR** | **Klinische Erwägungen:** | | | | | **(1)DC-** | **(2)antileuk** |
| | | GEPLANTE in vivo Applikationsart (AA), Dosierung (Do): angegeben als 'range' (Do^{R}) sowie im immunmodulatorischen Kontext/bezogen auf 6l Blut (Do^{I}), Verabreichungsfrequenz (VF)und -Dauer(VD) | | | | | **generierung s-effizienz N/NN (%)** | **äm. T-Zell-funktion N/NN (%)** |
| | | **AA** (fett: Geplant) | **Do^{R}** | **Do^{I}/[6l]** | **VF** | **VD** (pauschal: 3 Monate bis zu 2 Jahre), zunächst: | | |
| | | | ⁽¹⁾µg/m² | | | | | |
| | | | ⁽²⁾mio l.E/m² | | | | | |
| | | | ⁽³⁾µg/Tag | | | | | |
| | | | ⁽⁴⁾ mg/kg /Tag | | | | | |
| | | | ⁽⁵⁾µg/m²[Tag | | | | | |
| | | | ⁽⁶⁾µg/Woche | | | | | |
| | | | ⁽⁷⁾mg/m²/Tag | | | | | |
| **HS.I:** | | | | | | | | |
| GM-CSF | 800U/ml, | ic/sc/**iv** | 15-500⁽¹⁾ | 75⁽⁵⁾ | ca3-7x/wo | 7-42 Tage | 30/30 (100%) | 14/19 (74) |
| PICIBANIL | 10µg/ml | im,sc,**id** | 20-500⁽³⁾ | 50⁽⁶⁾ | ca3-7x/wo | -21 Tage | | |

| **HS-K:** | | | | | | | | |
|---|---|---|---|---|---|---|---|---|
| GM-CSF | 800U/ml, | ic/sc/**iv** | 15-500⁽¹⁾ | 75⁽⁵⁾ | ca3-7x/wo | 7-42 Tage | 13/13 (100%) | 7/9(78) |
| PGE₂ | 1 µg/ml, | iv,sc,im | 0.1-5⁽⁴⁾ | 0.36⁽⁴⁾ | ca3-7x/wo | 7-42 Tage | | |

| **HS-M:** | | | | | | | | |
|---|---|---|---|---|---|---|---|---|
| GM-CSF | 800U/ml, | ic/sc/**iv** | 15-500⁽¹⁾ | 75⁽⁵⁾ | ca3-7x/wo | 7-42 Tage | 3/4(75%) | 3/3 (100) |
| PGE₁ | 1 µg/ml, | iv | 0.075-500⁽³⁾ | 0.36⁽⁴⁾ | ca7x/wo | -30 Tage | | |

| **HS-D:** | | | | | | | | |
|---|---|---|---|---|---|---|---|---|
| GM-CSF | 800U/ml, | ic/sc/**iv** | 15-500⁽¹⁾ | 75⁽⁵⁾ | ca3-7x/wo | 7-42 Tage | 38/43 (88%) | 10/14 (71) |
| PICIBANIL | 10µg/ml, | im,sc,**id** | 20-500⁽³⁾ | 50⁽⁶⁾ | ca3-7x/wo | -21 Tage | | |
| PGE₂ | 1 µg/ml, | **iv**,sc,im | 0.1-5⁽⁴⁾ | 0.36⁽⁴⁾ | ca3-7x/wo | 7-42 Tage | | |

| **HS-F:** | | | | | | | | |
|---|---|---|---|---|---|---|---|---|
| **GM-CSF** | 800U/ml, | ic/sc/**iv** | 15-500⁽¹⁾ | 75⁽⁵⁾ | ca3-7x/wo | 7-42 Tage | 42/45(93%) | 11/14 (79) |
| CALCIMYCIN | 375ng/ml | ex vivo | 5-10 µM | 18(4) | ca3-7x/wo? | ca3-7x/wo? | | |

| **HS-A:** | | | | | | | | |
|---|---|---|---|---|---|---|---|---|
| GM-CSF | 800U/ml, | ic/sc/**iv** | 15-500 ⁽¹⁾ | 75⁽⁵⁾ | ca3-7x/wo | 7-42 Tage | 36/44 (82%) | 3/3 (100) |
| TNFα | 10ng/ml | **iv** | 0.04-4 ⁽⁷⁾ | 0.06⁽⁷⁾ | ca1-2x/wo | -56 Tage | | |

| **HS-E:** | | | | | | | | |
|---|---|---|---|---|---|---|---|---|
| **GM-CSF** | 800U/ml, | ic/sc/**iv** | 15-500 ⁽¹⁾ | 75⁽⁵⁾ | ca3-7x/wo | 7-42 Tage | 15/25 (60%) | 6/9 (67) |
| INTRON | 500U/ml | iv/sc/im | 2-5 ⁽²⁾ | 3-3.5⁽²⁾ | ca 3-7x/wo | -180 Tage | | |

| **HS-C:** | | | | | | | | |
|---|---|---|---|---|---|---|---|---|
| GM-CSF | 800U/ml, | ic/sc/**iv** | 15-500 ⁽¹⁾ | 75⁽⁵⁾ | ca3-7x/wo | 7-42 Tage | 30/37 (81%) | 5/9 (56) |
| TNFα | 10ng/ml, | **iv** | 0.04-4⁽⁷⁾ | 0.06⁽⁷⁾ | ca1-2x/wo | -56 Tage | | |
| PGE₂ | 1 µg/ml, | **iv**,sc,im | 0.1-5 ⁽⁴⁾ | 0.36⁽⁴⁾ | ca3-7x/wo | 7-42 Tage | | |

| **HS-G:** | | | | | | | | |
|---|---|---|---|---|---|---|---|---|
| GM-CSF | 800U | ic/sc/**iv** | 15-500 ⁽¹⁾ | 75⁽⁵⁾ | ca3-7x/wo | 7-42 Tage | 18/27 (66%) | |

| **HS-H:** | | | | | | | | |
|---|---|---|---|---|---|---|---|---|
| INTRON | 500U/ml | **iv**/sc/im | 2-5 (2) | 3-3.5⁽²⁾ | ca 3-7x/wo | -180 Tage | 4/12 (33%) | |

| **HS-L:** | | | | | | | | |
|---|---|---|---|---|---|---|---|---|
| PICIBANIL | 10µg/ml | im,sc,**id** | 20-500⁽³⁾ | 50⁽⁶⁾ | ca3-7x/wo | -21 Tage | 6/9 (67%) | |
| **w/o** | | | | | | | 6/41 (15%) | 16/22 (73) |

| | | | | | | | | |
|---|---|---|---|---|---|---|---|---|
| Bezogen auf 6 Liter Blut, entsprechend der in vitro verwendeten Wirkstoffkonzentrationen w/o WB Kontrolle ohne Kits | | | | | | | | |

## Patentansprüche

1. Wirkstoffkombination zur Anwendung in der parenteralen, individuellen Therapie und/oder Prävention der myeloischen Leukämie bei Patienten in Remission der Erkrankung, enthaltend GM-CSF und nur einen weiteren Wirkstoff, ausgewählt aus PICIBANIL, PGE₁, PGE₂, CALCIMYCIN und TNFa sowie pharmazeutisch verträgliche Hilfsstoffe, die Wasser, wässrige Lösungen, Puffer, Pufferlösungen, Bindemittel und/oder Adjuvantien umfassen, wobei die myeloische Leukämie eine akute myeloische Leukämie, myelodysplastische Erkrankung, myeloproliferative Erkrankung oder chronisch myeloische Leukämie ist.

2. Wirkstoffkombination nach Anspruch 1, wobei die Behandlung mit folgender Dosierung durchgeführt wird:
**GM-CSF** als intravenöse Dauerinfusion oder täglich wechselnd mit 15 bis 500 µg/Tag, vorzugsweise 75 µg/Tag, und **PICIBANIL** intramuskulär, intradermal oder subcutan mit 20 bis 500 µg/ Tag, vorzugsweise 50 µg /Tag, wobei GM-CSF und PICIBANIL in dieser Reihenfolge im täglichen, zweitägigen oder dreitägigen Abstand verabreicht werden.

3. Wirkstoffkombination nach Anspruch 1, wobei die Behandlung mit folgender Dosierung durchgeführt wird:
**GM-CSF** als intravenöse Dauerinfusion oder täglich wechselnd mit 15 bis 500 µg/Tag, vorzugsweise 75 µg/Tag, und **PGE₁** intravenöse oder als Dauerinfusion mit 0,075 µg /kg/h, bis maximal 500 µg/kg/h, wobei GM-CSF und PGE₁ in dieser Reihenfolge im täglichen, zweitägigen oder dreitägigen Abstand verabreicht werden.

4. Wirkstoffkombination nach Anspruch 1, wobei die Behandlung mit folgender Dosierung durchgeführt wird:
**GM-CSF** als intravenöse Dauerinfusion oder täglich wechselnd mit 15 bis 500 µg/Tag, vorzugsweise 75 µg/Tag, und **PGE₂** mit 0,1 bis 5 mg/kg/Tag,
wobei GM-CSF und PGE₂ in dieser Reihenfolge im täglichen, zweitägigen oder dreitägigen Abstand verabreicht werden.

5. Wirkstoffkombination nach Anspruch 1, wobei die Behandlung mit folgender Dosierung durchgeführt wird:
**GM-CSF** als intravenöse Dauerinfusion oder täglich wechselnd mit 15 bis 500 µg/Tag, vorzugsweise 75 µg/Tag, und **TNFα** intravenöse 0.04mg-4mg/m²/24h,
wobei GM-CSF und TNFα in dieser Reihenfolge im täglichen, zweitägigen oder dreitägigen Abstand verabreicht werden.

6. Wirkstoffkombination nach Anspruch 1, wobei die Behandlung mit folgender Dosierung durchgeführt wird :
**GM-CSF** als intravenöse Dauerinfusion oder täglich wechselnd mit 15 bis 500 µg/Tag, vorzugsweise 75 µg/Tag, und **CALCIMYCIN** Dosierung 0.1-10µg,
wobei GM-CSF und CALCIMYCIN in dieser Reihenfolge im täglichen, zweitägigen Abstand verabreicht werden.

7. Wirkstoffkombination nach einem der vorherigen Ansprüche zur parenteralen Verabreichung.

8. Verfahren zur Auswahl einer geeigneten Wirkstoffkombination zur individuellen Therapie und/oder Prävention der myeloischen Leukämie, mit folgenden Schritten:
(a) Unterteilung einer blastenhaltigen Vollblut-Probe eines Patienten in mehrere Unterproben;
(b) Ex vivo Isolation von T-Zellen aus einer Probe von Vollblut des Patienten oder eines Stammzellspenders;
(c) Sequenzielle Zugabe der folgenden einzelnen Wirkstoffkombinationen zu den einzelnen blastenhaltigen Vollblut-Unterproben jeweils zu Beginn sowie nach einer ersten Inkubationszeit der Unterproben von vorzugsweise 4 bis 6 Tagen, wobei durch die Wirkstoffkombination in den Unterproben leukämische Blasten zu leukämischen dendritischen Zellen DCleu umgewandelt werden:
(i) GM-CSF und PICIBANIL
(ii) GM-CSF und PGE₂
(iii) GM-CSF und PGE₁
(iv) GM-CSF und TNFα
(v) GM-CSF und CALCIMYCIN
(d) Zugabe der in Schritt (a) isolierten T-Zellen zu jeder der in Schritt (c) nach Inkubation erhaltenen Unterproben und anschließende Inkubation derselben während einer zweiten Inkubationszeit von vorzugsweise 7 bis 10 Tagen;
(e) Ausführung eines Lysetests in jeder der Unterproben nach der Inkubationszeit in Schritt (d) zum Nachweis der antileukämischen Funktion der jeweils verwendeten Wirkstoffkombination;
(f) Erstellen eines Rankings der verwendeten Wirkstoffkombinationen abhängig von der Güte des Ergebnisses der jeweiligen Lysetests; und
(g) Auswahl der Wirkstoffkombination nach der besten Umwandlung von Blasten in DCleu ohne Induktion der Blastenproliferation und dem besten Blastenlyseergebnis, erzielt durch antileukämische T-Zellaktivierung.

## Claims

1. Combination of active agents for administration in parenteral, individual therapy and/or prevention of myeloid leukemia in patients in remission of the disease, comprising GM-CSF and only one further agent selected from PICIBANIL, PGE1, PGE2, CALCIMYCIN and TNFα as well as pharmaceutically compatible excipients, which comprise water, aqueous solutions, buffers, buffer solutions, binders and/or adjuvants, wherein the myeloid leukemia is an acute myeloid leukemia, myelodysplastic disease, myeloproliferative disease or chronic myeloid leukemia.

2. Combination of active agents according to claim 1, wherein the treatment is carried out at the following dosage:
**GM-CSF** as intravenous continuous infusion or daily alternating with 15 to 500 µg/day, preferably 75 µg/day, and PICIBANIL intramuscularly, intradermally or subcutaneously with 20 to 500 µg/day, preferably 50 µg/day, wherein GM-CSF and **PICIBANIL** are administered in this order in daily, two-day or three-day intervals.

3. Combination of active agents according to claim 1, wherein the treatment is carried out at the following dosage:
**GM-CSF** as intravenous continuous infusion or daily alternating with 15 to 500 µg/day, preferably 75 µg/day, and **PGE1** intravenous or as continuous infusion with 0.075 µg /kg/h, up to a maximum of 500 µg/kg/h, GM-CSF and PGE1 being administered in this order in daily, two-day or three-day intervals.

4. Combination of active agents according to claim 1, wherein the treatment is carried out at the following dosage:
**GM-CSF** as intravenous continuous infusion or daily alternating with 15 to 500 µg/day, preferably 75 µg/day, and **PGE2** with 0.1 to 5 mg/kg/day,
wherein GM-CSF and PGE2 are administered in this order in daily, two-day or three-day intervals.

5. Combination of active agents according to claim 1, wherein the treatment is carried out at the following dosage:
**GM-CSF** as intravenous continuous infusion or daily alternating with 15 to 500 µg/day, preferably 75 µg/day, and **TNFα** intravenous 0.04mg-4mg/m2/24h,
GM-CSF and TNFa being administered in this order in daily, two-day or three-day intervals.

6. Combination of active agents according to claim 1, wherein the treatment is carried out at the following dose level:
**GM-CSF** as intravenous continuous infusion or daily alternating with 15 to 500 µg/day, preferably 75 µg/day, and **CALCIMYCIN** dosage 0.1-10µg,
where GM-CSF and CALCIMYCIN are administered in this order in daily or two-day intervals.

7. Combination of active agents according to one of the previous claims for parenteral administration.

8. Method for selecting a suitable combination of active agents for individual therapy and/or prevention of myeloid leukaemia, comprising the following steps:
(a) subdivision of a patient's whole blood sample containing blasts into several sub-samples;
(b) ex vivo isolation of T cells from a sample of whole blood from the patient or from a stem cell donor;
(c) sequential addition of the following individual combinations of active agents to the individual blast-containing whole blood sub-samples at the beginning and after a first incubation period of the sub-samples of preferably 4 to 6 days, wherein the combination of active agents converts in the sub-samples leukemic blasts to leukemic dendritic cells DCleu:
(i) GM-CSF and PICIBANIL
(ii) GM-CSF and PGE2
(iii) GM-CSF and PGE1
(iv) GM-CSF and TNFα
(v) GM-CSF and CALCIMYCIN
(d) addition of the T cells isolated in step (a) to each of the sub-samples obtained in step (c) after incubation and subsequently incubating them for a second incubation period of preferably 7 to 10 days;
(e) performing a lysis test in each of the sub-samples after the incubation period in step (d) to detect the antileukemic function of the combination of active agents used;
(f) establishing a ranking of the combinations of active agents used depending on the quality of the result of the respective lysis tests; and
(g) selection of the combination of active agents according to the best conversion of blasts into DCleu without induction of blasts' proliferation and the best blast lysis result achieved by antileukemic T cell activation.

## Revendications

1. Combinaison d'agents actifs pour l'application dans la thérapie parentérale et individuelle et/ou la prévention de la leucémie myéloïde chez des patients en rémission de la maladie, comprenant GM-CSF et seulement une autre substance choisie parmi PICIBANIL, PGE1, PGE2, CALCIMYCIN et TNFα ainsi que des excipients pharmaceutiquement acceptables, qui comprennent de l'eau, des solutions aqueuses, des tampons, des solutions tampons, des liants et/ou des adjuvants, la leucémie myéloïde étant une leucémie myéloïde aigue, une maladie myélodysplasique, une maladie myéloproliférative ou une leucémie myéloïde chronique.

2. Combinaison d'agents actifs selon la revendication 1, le traitement étant effectué avec le dosage suivant:
**GM-CSF** en perfusion intraveineuse continue ou en alternance quotidienne avec 15 à 500 µg/jour, de préférence 75 µg/jour, et **PICIBANIL** par voie intramusculaire, intradermique ou sous-cutanée avec 20 à 500 µg/jour, de préférence 50 µg/jour, où GM-CSF et PICIBANIL sont administrés dans cet ordre à intervalles d'un jour, de deux jours ou de trois jours.

3. Combinaison d'agents actifs selon la revendication 1, le traitement étant effectué avec le dosage suivant:
**GM-CSF** en perfusion intraveineuse continue ou en alternance quotidienne avec 15 à 500 µg/jour, de préférence 75 µg/jour, et **PGE1** en perfusion intraveineuse ou en perfusion continue avec 0,075 µg /kg/h, jusqu'à un maximum de 500 µg/kg/h, GM-CSF et PGE1 étant administrés dans cet ordre à intervalles d'un jour, de deux jours ou de trois jours.

4. Combinaison d'agents actifs selon la revendication 1, le traitement étant effectué avec le dosage suivant:
**GM-CSF** en perfusion intraveineuse continue ou en alternance quotidienne avec 15 à 500 µg/jour, de préférence 75 µg/jour, et **PGE2** avec 0,1 à 5 mg/kg/jour,
dans laquelle GM-CSF et PGE2 sont administrés dans cet ordre à intervalles d'un jour, de deux jours ou de trois jours.

5. Combinaison d'agents actifs selon la revendication 1, le traitement étant effectué avec le dosage suivant:
**GM-CSF** en perfusion intraveineuse continue ou quotidienne en alternance avec 15 à 500 µg/jour, de préférence 75 µg/jour, et **TNFα** intraveineuse 0,04mg-4mg/m2/24h,
GM-CSF et TNFα étant administrés dans cet ordre à intervalles d'un jour, de deux jours ou de trois jours.

6. Combinaison d'agents actifs selon la revendication 1, le traitement étant effectué avec le dosage suivant:
**GM-CSF** en perfusion intraveineuse continue ou en alternance quotidienne avec 15 à 500 µg/jour, de préférence 75 µg/jour, et **CALCIMYCIN** dose 0.1-10µg,
où GM-CSF et CALCIMYCIN sont administrés dans cet ordre à des intervalles quotidiens ou de deux jours.

7. Combinaison d'agents actifs selon l'une des revendications précédentes pour l'administration parentérale.

8. Procédé de sélection d'une combinaison d'agents actifs appropriée pour le traitement individuel et/ou la prévention de la leucémie myéloïde, comprenant les étapes suivantes:
(a) subdivision d'un échantillon de sang total d'un patient contenant des blastes en plusieurs sous-échantillons;
b) isolement ex vivo de cellules T à partir d'un échantillon de sang total du patient ou d'un donneur de cellules souches;
(c) ajout séquentiel des combinaisons d'agents actifs suivantes aux sous-échantillons individuels de sang total contenant des blastes au début et après une première période d'incubation des sous-échantillons de préférence de 4 à 6 jours, dans lequel la combinaison de substances convertit dans les sous-échantillons les blastes leucémiques en cellules dendritiques leucémiques CDleu:
i) GM-CSF et PICIBANIL
ii) GM-CSF et PGE2
iii) GM-CSF et PGE1
(iv) GM-CSF et TNFα
v) GM-CSF et CALCIMYCIN
(d) addition des cellules T isolées à l'étape (a) à chacun des sous-échantillons obtenus après incubation à l'étape (c), suivi d'une incubation des mêmes pendant une seconde période d'incubation de préférence de 7 à 10 jours ;
(e) effectuer un test de lyse dans chacun des sous-échantillons après la période d'incubation de l'étape (d) pour montrer la fonction antileucémique de la combinaison d'agents actifs utilisée;
(f) établir un classement des combinaisons d'agents actifs utilisés en fonction de la qualité des résultats des tests de lyse respectifs; et
(g) sélection de la combinaison d'agents actifs en fonction de la meilleure conversion des blastes en DCleu sans induction de la prolifération des blastes et du meilleur résultat de lyse des blastes, obtenu par activation des cellules T antileucémiques.
